# EUROPÄISCHE PATENTANMELDUNG

(11) **EP 1 359 158 A2**
(43) Veröffentlichungstag der Anmeldung: **05.11.2003**
(21) Anmeldenummer: 03090081.5
(22) Anmeldetag: 25.03.2003
(51) Int. Cl.: C07K 14/47, C12N 15/12, C12Q 1/68, G01N 33/68

(54) **Trp-p8 Splice Varianten und regulatorische RNA**

(30) Priorität: 02.04.2002 DE 10215321
(71) Anmelder: metaGen Pharmaceuticals GmbH, 13347 Berlin (DE)
(72) Erfinder: Kaiser, Simone, 13347 Berlin (DE); Plath, Thomas, 13347 Berlin (DE)
(74) Vertreter: Jungblut, Bernhard Jakob, Dr.

(57) **Zusammenfassung**

Die Erfindung betrifft neue Trpp8 Sequenzen sowie deren Verwendungen im Zusammenhang mit der Diagnose und/oder Behandlung von Krebs.

## Beschreibung

### Gebiet der Erfindung

Die Erfindung betrifft neue Trp-p8 (transient receptor potential protein 8, folgend kurz Trpp8) Splice Varianten und regulatorische RNA sowie hierdurch codierte Proteine bzw. Peptide, die Verwendung von aus Trpp8 abgeleiteten Substanzen zum Screenen nach daran bindenden Substanzen, sowie die Verwendung von an Trpp8 bindenden Substanzen zur Diagnose und/oder Behandlung von Krebserkrankungen.

### Hintergrund der Erfindung und Stand der Technik

Die Kalzium Homeostase regelt wichtige Zellfunktionen, wie Proliferation, Differenzierung, Invasion, Migration, Angiogenese und Apoptose. Bei Prostatakrebs spielt Kalzium eine wichtige Rolle in der Tumorbildung. Es ist jedoch wenig über die Kalziumkanäle und membrangebundenen Plasma Rezeptoren bekannt, die den Eintritt und Austritt von Kalzium in und aus intrazellulären Kalziumreservoirs in Prostatatumorzellen regeln.

Trpp8 ist in der Literaturstelle Tsavaler et al., Cancer Res, 61:3760-3769 (2001) als Prostata-spezifisches Gen beschrieben worden, welches vorwiegend in humanen Prostatatumoren exprimiert wird. Trpp8 wird signifikant hochreguliert. In Androgen-abhängigen Prostata Zelllinien wird gemäß dieser Literaturstelle Trpp8 gefunden, nicht jedoch in Androgen-unabhängigen Zelllinien, welche auch nicht PAP (prostate acid phosphatase) und PSA (prostate specific antigen) exprimieren. Es wird vermutet, daß Trpp8 als Kalzium Kanal Protein funktioniert. Nach neuer Nomenklatur der Trp-Superfamilie wird Trpp8 zukünftig auch als TRPM8 bezeichnet (Montell et al., Mol Cell, 9, 229-231, 2002).

Trp Proteine sollen zu den sogenannten store operated calcium channels (SOC) bzw. capacitative calcium entry channels (CCE) gehören. In LNCaP Zellen konnte eine Involvierung in der Apoptose gezeigt werden (Wertz et al., J Biol Chem, 275:11470-11477 (2000)).

Die 5694 bp Trpp8 cDNA hat einen 3312 bp offenen Leserahmen, welcher für ein 1104 Aminosäuren Protein mit angeblich sieben transmembranen Domänen codiert mit einem Molekulargewicht von ca. 127.500 Da. Unveröffentlichte bioinformatische Analyse der Anmelderin legen jedoch nahe, daß Trpp8 lediglich 6 transmembrane Domänen aufweist.

Trpp8 Sequenzen sind in den Literaturstellen US-6,194,152, US-6,183,968, WO-99/46374, WO-99/09166, WO-01/25273, WO-01/25272, WO-01/34802, WO-01/46258, WO-01/42467 und WO-01/1633 beschrieben. Die Literaturstellen US-6,194,152 und WO-01/51633 offenbaren die Verwendung der darin genannten Sequenzen zur Detektion von Tumorzellen sowie verschiedener Substanzklassen in allgemeiner Weise zur Behandlung von Prostatakrebs.

Prostatakrebs ist eine mit zunehmendem Alter mit beachtlicher Inzidenz auftretende Erkrankung. Bislang wird Prostatakrebs im wesentlichen pathologisch diagnostiziert und meist durch Entfernung der Prostata behandelt. Die Entfernung der Prostata hat verschiedene nachteilige Effekte auf einen Patienten. Eine verbesserte Diagnose und Behandlung dieser Krebsart, insbesondere ohne das Erfordernis einer Entfernung der Prostata, ist daher in hohem Maße wünschenswert.

### Technisches Problem der Erfindung

Der Erfindung liegt das technische Problem zugrunde, pharmazeutische Zusammensetzungen zur Diagnose und/oder zur Behandlung von Prostatakrebs-Erkrankungen anzugeben.

Grundzüge der Erfindung sowie bevorzugte Ausführungsbeispiele.

Zur Lösung dieses technischen Problems lehrt die Erfindung zunächst eine Nukleinsäure codierend für eine Trpp8 Splice Variante oder regulatorische RNA enthaltend eine Nukleinsäuresequenz gemäß einer der Sequenzen Seq.-ID 1 bis 7 oder 23 bis 31 oder 32 bis 38, sowie ein Trpp8 Peptid oder Protein enthaltend eine Aminosäuresequenz codiert durch eine der Nukleinsäuresequenzen Seq.-ID 1 bis 7 oder 23 bis 30 oder gemäß Seq.-ID 8 oder 39 bis 59, wobei bekannte Sequenzen enthaltend diese Sequenzen ausgeschlossen sind. Die Nukleinsäure bzw. das Peptid oder Protein können insbesondere aus diesen Sequenzen bestehen. Erfindungsgemäße Nukleinsäuren oder Proteine bzw. Peptide lassen sich mit den üblichen molekularbiologischen Methoden herstellen.

Die Splice Variante (6b) gemäß Seq.-ID 1 enthält 6 Exons mit einer Länge von 985 Basenpaaren. Exons 1 bis 5 sind identisch mit bekanntem Vollängen Trpp8. Exon 6 is um 234 Basenpaare verlängert und geht in das poly-A-Signal über. Eine Teilsequenz ist in Seq.-ID 23 angegeben.

Die Splice Variante (16b) gemäß Seq.-ID 2 enthält zumindest 2 Exons mit einem unbekannten 5'-Ende. Exon 15 ist identisch mit bekanntem Trpp8. Das erfindungsgemäße Exon 16b schließt sich mit 104 Basenpaaren unmittelbar an Exon 16 an und geht in ein poly-A-Signal über. Eine Teilsequenz ist in Seq.-ID 24 angegeben.

Die Splice Variante (20b) gemäß Seq.-ID 3 enthält zumindest 8 Exons mit einem unbekannten 3'-Ende. Exon 20b schließt sich mit 127 Basenpaaren unmittelbar an Exon 20 an. Exon 19 bis 26 sind identisch zu Trpp8, allerdings endet das Exon 26 schon nach 1136 Basen mit einem Poly-A-Signal. Eine Teilsequenz ist in Seq.-ID 25 angegeben.

Die Splice Variante (avant25) gemäß Seq.-ID 4 enthält zumindest 3 Exons mit einem unbekannten 5'-Ende. Avant25 weist 571 Basenpaare auf, deren Sequenz in bekanntem Trpp8 nicht gefunden wird. Die Sequenz befindet sich im Intron-Bereich zwischen Exon 24 und 25 von Trpp8. Die Sequenz geht weiter in Exon 25 und 26 von Trpp8. Exon 26 ist wie bei 20b abgebrochen. Das poly-A-Signal beginnt beim Basenpaar 1136. Eine Teilsequenz ist in Seq.-ID 26 angegeben.

Die Splice Variante (avant13) gemäß Seq.-ID 5 enthält zumindest 5 Exons mit einem unbekannten 5'-Ende. Avant13 weist ein Exon von 271 Basenpaaren im Intronbereich von Trpp8 zwischen dessen Exon 12 und 13 auf. Dieses Exon geht in Exon 21 über, wobei lediglich 20 Basenpaare des 3'-Endes Teil des Transkrips sind. Die Exons 22, 23, 24, und 26 sind identisch mit der Vollänge von Trpp8, weisen aber ein anderes Splice Muster auf. Exon 25 fehlt. Exon 26 ist abgebrochen und enthält ein poly-A-Signal beginnend beim Basenpaar 1136. Teilsequenzen sind Seq.-ID 27 und 31.

Die Splice Variante (4a_4b) gemäß Seq.-ID 6 enthält zumindest 5 Exons. Exon 2x ist ein separates Exon, welches sich im Intron-Bereich von Trpp8 zwischen Exon 2 und 3 befindet und aus mindestens 35 Basenpaaren besteht (das 5'-Ende von Exon 2x ist nicht komplett), und mehrere hundert Basen vor Exon 3 gemäß bekanntem Trpp8 angeordnet. Exon 3 wird wie in Trpp8 transkribiert, aber Exon 4 startet 46 Basen früher als in Trpp8. Die Sequenz setzt sich über Exon 5 zu 6a fort und endet mit 6b. Teilsequenzen sind in Seq.-ID 28 - 30 angegeben.

Die Splicevariante (16b-lang) gemäß Seq.-ID 32 enthält 16 Exons. Exon 1 bis 15 sind identisch mit Trpp8, das erfindungsgemäße Exon 16b schliesst sich mit 104 Basenpaaren unmittelbar an Exon 16 an und geht in ein Poly-A-Signal über.

Die Splicevariante (16b-Unter-Splice-A) gemäß Seq.-ID 33 besteht aus mindestens 3 Exons mit unbekanntem 5'-Ende. Die Sequenz beginnt in Exon 12 von Trpp8, geht in Exon 13 über allerdings nur mit 22 Basenpaaren, dann geht es in Exon 16 über ab Base 22 (kryptisches Splicen), welches dann nach 83 Basen in ein Poly-A-Signal übergeht.

Die Splicevariante (16b-Unter-Splice-B) gemäß Seq.-ID 34 besteht aus mindestens 4 Exons mit unbekanntem 5'-Ende. Die Sequenz beginnt in Exon 12 von Trpp8, endet aber um 67 Basen vorzeitig in Exon 12 und geht in Exon 14 ab Base 71 über (kryptisches Splicen). Von Exon 14 geht die Sequenz in Exon 15, 16 und 16b über und endet mit einem Poly-A-Signal.

Die Splicevariante (16b-Unter-Splice-C) gemäß Seq.-ID 35 besteht aus mindestens 4 Exons mit unbekanntem 5'-Ende. Die Sequenz beginnt in Exon 12 von Trpp8, geht in Exon 13, 14, 16 und 16b mit in allen Fällen kanonischem Splicen über und endet mit einem Poly-A-Signal.

Die Splicevariante (16b-Unter-Splice-D) gemäß Seq.-ID 36 besteht aus mindestens 4 Exons. Die Sequenz beginnt in Exon 9 von Trpp8, geht in Exon 10 über, endet aber kanonisch nach 53 Basen. Die Sequenz geht weiter in den letzten 11 Basen von Exon 15, dann in Exon 16 und 16b über und endet mit einem Poly-A-Signal.

Die Splicevariante (20b-lang) gemäß Seq.-ID 37 enthält 26 Exons. Exon 1 bis 19 und 21 bis 25 sind identisch mit Trpp8, das erfindungsgemäße Exon 20b schließt sich mit 127 Basenpaaren unmittelbar an Exon 20 an. Exon 26 ist gegenüber Trpp8 verkürzt, das Poly-A-Signal beginnt beim Basenpaar 1136.

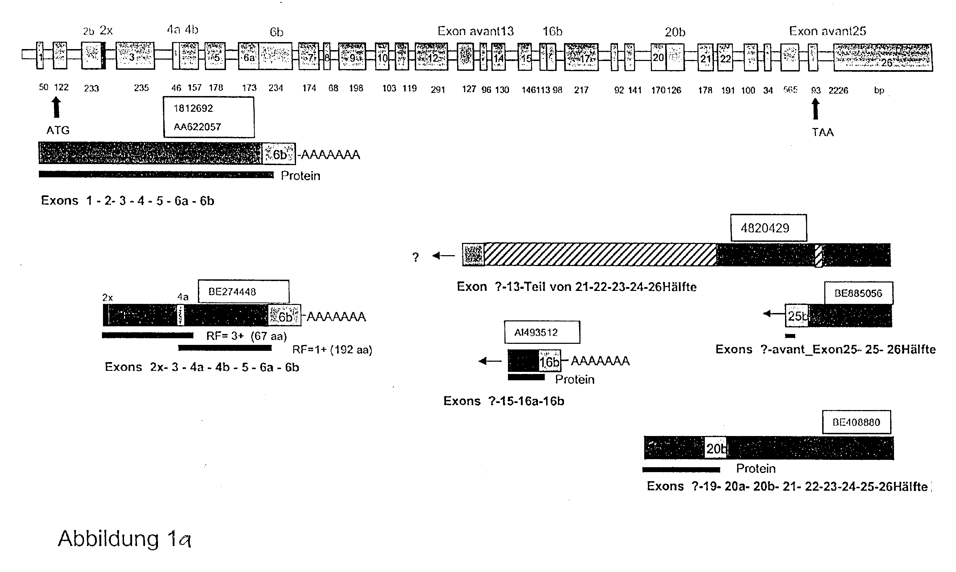

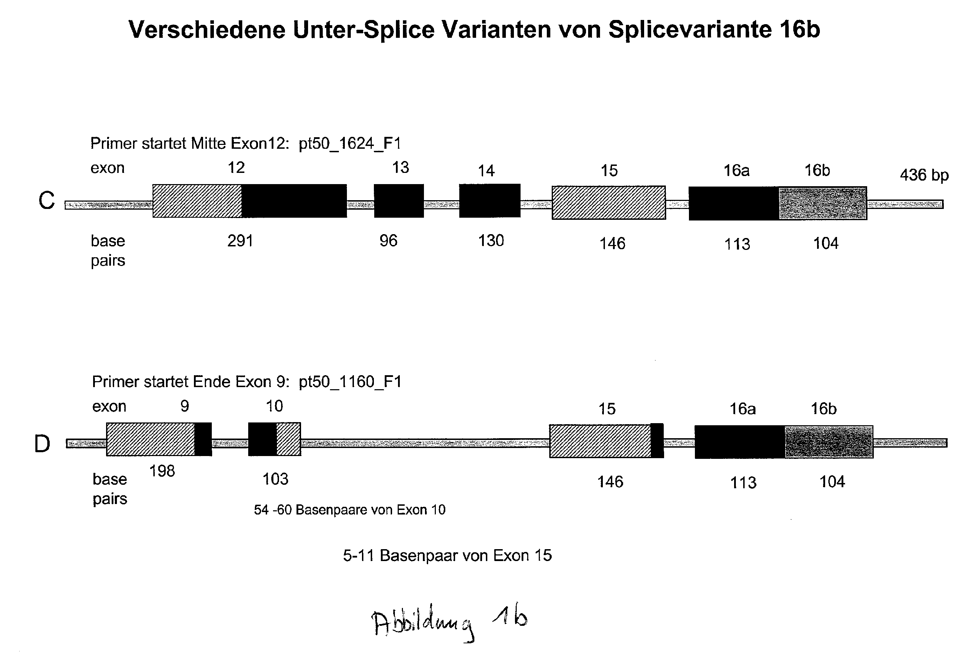

Im Einzelnen wird auch auf die graphische Darstellung der jeweiligen Lagen gemäß Abbildung 1a und 1b hingewiesen.

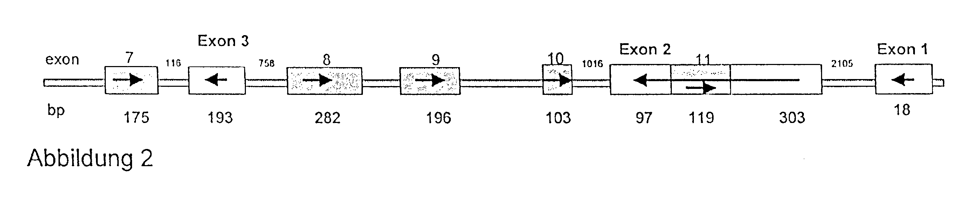

Das neue regulatorische Trpp8 Gen gemäß der Sequenzen Seq.-ID 7 und 8 bzw. 38 und 52 bis 56 enthält 3 Exons und ist auf dem gegenüberliegenden Strang des bekannten Trpp8 Gens auf Chromosom 2 angeordnet. Exon 1 des regulatorischen Gens liegt im Intron zwischen Exon 12 und 11 des Trpp8. Exon 2 des neuen Gens umfaßt Exon 11 von Trpp8 vollständig. Exon 3 des neuen Gens ist zwischen Exon 8 und 7 des Trpp8 angeordnet. Erfindungsgemäße mRNA bindet möglicherweise bekanntes Trpp8 oder seine Splice Varianten und beeinflußt deshalb vermutlich die Expression dieser Gene. Diese Bindung könnte eine Stabilisierung und somit eine länger andauernde Translation induzieren, könnte aber auch eine Destabilisierung der mRNA durch Aktivierung der Abbaumechanismen bewirken. Insofern betrifft die Erfindung auch ein Verfahren zur Modulation der Trpp8 Expression, insbesondere deren Destabilisierung, wobei einer Zielzelle, beispielsweise Prostatatumorzelle, eine Substanz enthaltend zumindest eine Teilsequenz aus oder die Vollsequenz gemäß Seq.-ID 7 oder 8 bzw. 38 oder 52 bis 59 in einer physiologisch wirksamen Dosis zugeführt wird. Damit eignen sich solche Substanzen auch zur Herstellung von pharmazeutischen Zusammensetzungen zur Behandlung von Krebs, insbesondere Prostatakrebs. Ansonsten geltend die zu den anderen Erfindungsgegenständen getroffenen Ausführungen analog. Die Lokalisierung der regulatorischen RNA ist in der Abb. 2 im Einzelnen dargestellt.

Die Sequenzen Seq.-ID 39 bis 56 sind Protein- bzw. Peptidsequenzen der vorstehend beschriebenen DNA Sequenzen. Die unterstrichenen Bereiche sind verschieden von Trpp8 und insbesondere erfindungsgemäße Sequenzen bzw. Teilsequenzen.

Die Sequenzen Seq.-ID 57 bis 59 sind beispielsweise zur Generierung von Antikörpern geeignet.

Die Erfindung betrifft weiterhin verschiedene Verwendungen der neuen Nukleinsäuren bzw. Peptide oder Protein, ebenso wie (gleiche) Verwendungen bereits bekannter Trpp8 Nukleinsäuren. Dies sind:
i) Die Verwendung einer für Trpp8 codierenden Nukleinsäure und/oder eines Trpp8 Peptids oder Proteins zur Detektion von Prostatakrebs oder zur Detektion eines Risikos der Erkrankung an Prostatakrebs, wobei eine Prostata-Gewebeprobe auf Übertranskription von Trpp8 RNA oder auf Überexpression eines Trpp8 Proteins untersucht wird, wobei vorzugsweise eine an für Trpp8 codierende Nukleinsäure oder eine an Trpp8 Protein oder Peptid bindende Detektorsubstanz, vorzugsweise enthaltend eine Reportergruppe, verwendet wird, wobei Bindung besagter Nukleinsäure und/oder besagten Proteins oder Peptids an die Detektorsubstanz halbquantitativ oder quantitativ detektiert wird,
ii) die Verwendung einer Trpp8 RNA oder eines Trpp8 Proteins oder Peptids zum Screenen nach daran bindenden Substanzen, insbesondere prospektiven Wirkstoffen zur Inhibierung von besagter RNA oder besagtem Protein oder Peptid, oder prospektiven Detektorsubstanzen, wobei eine prospektive Substanz oder eine Mischung solcher prospektiver Substanzen mit besagter RNA oder besagtem Protein oder Peptid kontaktiert wird, wobei mit einem Bindungsassay Bindungsereignisse festgestellt werden, und wobei eine bindende prospektive Substanz, ggf. nach Dekonvolutierung, selektiert wird,
iii) die Verwendung einer Trpp8 inhibierenden oder daran bindenden Substanz zur Herstellung einer pharmazeutischen Zusammensetzung zur Behandlung von Prostatakrebs.

Die bindende Substanz kann ein Antikörper sein, welcher durch Immunisierung eines nicht-menschlichen Säugetiers mit einer Trpp8 cDNA oder mit einem Trpp8 Peptid oder Protein erhältlich ist. Die Substanz kann aber auch eine Mimikryverbindung eines Antikörpers gegen ein Trpp8 Peptid oder Protein, sein. Die Substanz kann schließlich ein Aptamer, eine antisense RNA eine RNA; oder ein Ribozym sein. Die 5 Substanz kann zusätzlich eine zytotoxische und/oder immunstimulierende Komponente tragen bzw. daran gebunden sein. Die pharmazeutische Zusammensetzung kann zur lokalen Applikation in Tumorzellen enthaltendem Tumoren hergerichtet sein.

Die Erfindung betrifft weiterhin ein Verfahren zur Diagnose einer Krebserkrankung, wobei eine erfindungsgemäße Detektorsubstanz in der Ausführungsform mit einer Reportergruppe in zu untersuchendes Gewebe (welches im Organismus verblieben oder daraus entnommen sein kann) appliziert wird, wobei das zu untersuchende Gewebe dann einer Detektionsverfahrenstufe unterworfen wird, welche sensitiv für die Reportergruppe ist, und wobei im Fall der Detektion eines definierten Mindestwertes der Reportergruppe im Gewebe das Gewebe als Tumorzellen enthaltend qualifiziert wird, sowie ein Verfahren zur Behandlung einer Prostatakrebs-Erkrankung, wobei eine erfindungsgemäße pharmazeutische Zusammensetzung in einer physiologisch wirksamen Dosis und üblicher galenischer Herrichtung einem Patienten dargereicht wird. Es versteht sich, daß ggf. zur Entfernung nicht gebundener Detektorsubstanz notwendige Waschverfahrensstufen zwischengeschaltet werden.

Die Erfindung beruht auf der Erkenntnis, daß Trpp8, insbesondere die erfindungsgemäßen Splice Varianten und regulatorische RNA differentiell in Prostatatumorgewebe exprimiert wird, i.e. in Prostatatumorgewebe ist die Expression höher, verglichen mit normalen Zellen gleichen Gewebes.

Dies erlaubt es einerseits, insbesondere diese neuen Varianten als Marker zur Identifizierung von Tumorzellen in der Prostata zu nutzen. Auf der anderen Seite bietet die Inhibierung von Trpp8 bzw. der Varianten, insbesondere auch bei lokaler Applikation, die Möglichkeit, in die Prostatatumor-spezifischen Trpp8 Assoziationen mit anderen Prozessen in den Tumorzellen einzugreifen und somit letztendlich den Tumorzell-spezifisch veränderten Stoffwechsel zu stören und zu einem Absterben oder zumindest einer Wachstumshemmung der Prostatatumorzellen beizutragen.

Im Rahmen der Erfindung kann es sich empfehlen, im Vorfeld einer Behandlung mit einer erfindungsgemäßen pharmazeutischen Zusammensetzung eine Probe aus einem Gewebe, welches als Tumorgewebe mit anderen Methoden identifiziert ist, zu entnehmen und die Gewebeprobe auf Expression bzw. Überexpression von Trpp8 bzw. der Varianten zu untersuchen. Alternativ kann mit einer erfindungsgemäßen Detektorsubstanz zur Diagnose in vivo auf Trpp8 Abhängigkeit getestet werden. Wird eine Expression bzw. Überexpression von Trpp8 gegenüber Normalgewebe gleichen Typs festgestellt, so ist die Anwendung der erfindungsgemäßen pharmazeutischen Zusammensetzung indiziert.

Handelt es sich bei dem Tumor um einem Typus, bei welchem Tumorzellen Trpp8 bzw. eine der Varianten exprimieren, Normalzellen gleichen Gewebetyps jedoch nicht oder nur schwach, so ist es besonders bevorzugt, wenn die an Trpp8 bzw. die Variante bindende Substanz zusätzlich eine zytotoxische, radioaktive und/oder immunstimulierende Komponente trägt. Dies führt dann letztendlich dazu, dass praktisch ausschließlich Tumorzellen getötet, sei es durch die Zytotoxizität, sei es durch Angriff durch das stimulierte Immunsystem, werden, während Normalzellen in dem Gewebe praktisch vollständig erhalten bleiben. In dieser Ausführungsform braucht die bindende Substanz selbst nicht inhibierend auf Trpp8 bzw. die Varianten zu wirken, da die bindende Substanz dann lediglich als Marker funktionieren muß, welcher die Komponenten zu Ziel-Tumorzellen trägt. Im Falle des Einsatzes einer zytotoxischen, radioaktiven und/oder immunstimulierenden Komponente kann es sich besonders empfehlen, wenn die pharmazeutische Zusammensetzung zur lokalen Applikation in Tumorzellen enthaltendem Gewebe hergerichtet ist, beispielsweise zur Injektion.

### Definitionen.

Im Rahmen dieser Beschreibung wird die Bezeichnung TRPP8 als Oberbegriff für alle humanen Isoformen, Splice Varianten und regulatorische RNA, bekannt oder neu, auf Nukleinsäuren- oder Aminosäurenbasis, verwendet. Mit diesen Begriffen mit umfaßt sind auch die ggf. im Rahmen dieser Beschreibung offenbarten kurzen Sequenzen, welche aus den verschiedenen Varianten stammen, beispielsweise Immunisierungssequenzen. Weiterhin mit umfaßt sind auch Homologe, wobei die Homologie zumindest 80%, vorzugsweise mehr als 90%, höchstvorzugsweise mehr als 95%, beträgt (berechnet mit dem Programm MEGALIGN, DNASTAR LASERGENE, in der zum nmeldezeitpunkt gültigen Fassung). Im Falle der Nukleinsäuresequenzen sind auch komplementäre oder allelische Varianten mit umfaßt. Weiterhin sind Sequenzen umfaßt, welche lediglich Teilsequenzen, beispielsweise ein Exon oder mehrere Exons, der explizit offenbarten Sequenzen oder komplementärer Sequenzen hierzu darstellen, mit der Maßgabe, daß diese Teilsequenzen im Falle der Nukleinsäuren eine für eine Hybridisierung mit einer erfindungsgemäßen Nukleinsäure hinreichende Länge, zumindest 50 oder 150 Basen, aufweisen und im Falle der Proteine bzw. Peptide, ggf. codiert durch eine Nukleinsäure, mit zumindest gleicher Affinität an ein protein- oder peptidspezifisches Zielmoleküle binden. Weiterhin sind alle mit erfindungsgemäß eingesetzten Nukleinsäuren hybridisierende Nukleinsäuren umfasst, nämlich solche, die unter stringenten Bedingungen (5°C bis 25°C unterhalb der Aufschmelztemperatur; siehe ergänzend J.M. Sambrook et al., A laboratory manual, Cold Spring Harbor Laboratory Press (1989) und E.M. Southern, J Mol Biol, 98:503ff (1975)) hybridisieren. Es versteht sich, daß die Erfindung auch Expressionskassetten umfaßt, i.e. eine oder mehrere der erfindungsgemäßen Nukleinsäuresequenzen mit mindestens einer Kontroll- oder regulatorischen Sequenz. Eine solche Expressionskassette kann auch eine Sequenz für ein bekanntes Protein umfassen, wobei im Zuge der Translation ein Fusionsprotein aus einem bekannten Protein und einem erfindungsgemäßen Protein oder Peptid entsteht. Ebenso sind auch antisense Sequenzen zu den vorstehenden Nukleinsäuresequenzen umfaßt. Mit umfaßt sind auch Expressionsvektoren enthaltend erfindungsgemäße Nukleinsäuren sowie damit transformierte Wirtszellen. Schließlich sind RNA sowie damit korrelierende DNA und umgekehrt umfaßt, ebenso wie genomische DNA als auch korrelierte cDNA und umgekehrt.

Im Zusammenhang mit erfindungsgemäßen Verwendungen umfassen die Begriffe der Nukleinsäuren oder Proteine bzw. Peptide neben den Volllängen der offenbarten Sequenzen (siehe auch vorstehender Absatz) auch Teilsequenzen hieraus, und zwar mit einer Mindestlänge von 12 Nukleotiden, vorzugsweise einer Mindestlänge von 30 bis 90 Nukleotiden, im Falle der Nukleisäuren und einer Mindestlänge von 4 Aminosäuren, vorzugsweise einer Mindestlänge von 10 bis 30 Aminosäuren, im Falle der Proteine oder Peptide. Insbesondere zwischen 12 und 90 sowie 4 und 30 ist jeder einzelne Zahlenwert als Mindestlänge möglich.

Die Begriffe der Detektion und/oder der Behandlung umfassen optional auch die Detektion und/oder Behandlung von Metastasen aus Primärtumoren in sonstigen Geweben. Der Begriff der Behandlung umfasst auch die Prophylaxe sowie die Nachbehandlung (bei nicht mehr detektierbarem Tumor oder bei stabilem Tumor). Der Begriff der Prophylaxe umfasst im Zusammenhang mit der Detektion auch die Vorsorgeuntersuchung.

Als Inhibitor ist eine Verbindung oder Substanz bezeichnet, welche entweder die Bildung von TRPP8 inhibiert oder gebildetes TRPP8 in der Aktivität reduziert, bezogen auf die TRPP8 in vitro oder in vivo Aktivität in Abwesenheit des Inhibitors. Insofern kann ein Inhibitor einerseits eine Substanz sein, welche in der Entstehungskaskade von TRPP8 inhibierend eingreift. Auf der anderen Seite kann ein Inhibitor eine Substanz sein, welche mit gebildetem TRPP8 eine Bindung eingeht, und zwar dergestalt, dass weitere physiologische Wechselwirkungen mit endogenen Substanzen zumindest reduziert sind. Als Inhibitoren können auch Moleküle dienen, die die Transkription des Zielgens beeinflussen und inhibieren. Solche Moleküle können Polyamide oder Zinkfingerproteine sein, die durch Bindung an DNA-Regionen der basalen Transkriptionsmaschinerie die Transkription verhindern. Die Transkription kann indirekt auch über die Inhibierung von Transkriptionsfaktoren geschehen, die für die Transkription des Zielgens essentiell sind. Die Inhibierung solcher Transkriptionsfaktoren kann über die Bindung an sogenannte Decoy-Aptamere gewährleistet werden.

Mimikry-Moleküle sind Verbindungen, die den variablen Bereich, insbesondere den Bindungsbereich eines Antikörpers nachbilden und an gleicher Stelle eines Zielmoleküls binden wie der zu Grunde liegende Antikörper. Desweiteren fallen Anticaline und Affibodies unter den Begriff der Mimikrimoleküle.

Der Begriff der Antikörper umfaßt humane, humanisierte und nicht-humanisierte polyklonale oder monoklonale Antikörper (typischerweise in vivo hergestellt). Der Begriff umfasst desweiteren Phage-Display-Antikörper Ribozym-Display Antikörper (kovalente Fusion zwischen RNA und Protein) und RNA-Display Antikörper (in vitro hergestellt). Der Begriff umfasst auch Antikörper, welche durch Chimerisierung, Humanisierung oder De-Imunisierung (Ausschneiden von T-Zellepitopen aus dem humanen Antikörper, die unerwünschte Immunreaktionen auslösen) modifiziert sind, sowie spezifische Fragmente der leichten und/oder der schweren Kette des variablen Bereiches zu Grunde liegender Antikörper vorstehender Art. Die Herstellung bzw. Gewinnung solcher Antikörper mit vorgegebenen Immunogenen ist dem Durchschnittsfachmann wohl vertraut und braucht nicht näher erläutert zu werden. Weiterhin umfaßt sind bispezifische Antikörper, welche einerseits an ein Auslösemolekül einer Immun-Effektorzelle (z.B. CD3, CD16, CD64) und andererseits an ein Antigen der Tumorzielzelle binden. Dies bewirkt letzendlich im Bindungsfall, daß die Tumorzelle getötet wird.

Die galenische Herrichtung einer erfindungsgemäßen pharmazeutischen Zusammensetzung kann in fachüblicher Weise erfolgen. Als Gegenionen für ionische Verbindungen kommen beispielsweise Na⁺, K⁺, Li⁺ oder Cyclohexylammonium infrage. Geeigente feste oder flüssige galenische Zubereitungsformen sind beispielsweise Granulate, Pulver, Dragees, Tabletten, (Mikro-) Kapseln, Suppositorien, Sirupe, Säfte, Suspensionen, Emulsionen, Tropfen oder Lösungen zur Injektion (i.v., i.p., i.m., s.c.) oder Vernebelung (Aerosole), transdermale Systeme, sowie Präparate mit protrahierter Wirkstoff-Freigabe, bei deren Herstellung übliche Hilfsmittel wie Trägerstoffe, Spreng-, Binde-, Überzugs-, Quellungs-, Gleit- oder Schmiermittel, Geschmacksstoffe, Süßungsmittel und Lösungsvermittler, Verwendung finden. Als Hilfsstoffe sei Magnesiumcarbonat, Titandioxid, Lactose, Mannit und andere Zucker, Talcum, Milcheiweiß, Gelatine, Stärke, Zellulose und ihre Derivate, tierische und pflanzliche Öle wie Lebertran, Sonnenblumen-, Erdnuss- oder Sesamöl, Polyethylenglycole und Lösungsmittel, wie etwa steriles Wasser und ein- oder mehrwertige Alkohole, beispielsweise Glycerin, genannt. Eine erfindungsgemäße pharmazeutische Zusammensetzung ist dadurch herstellbar, dass mindestens ein erfindungsgemäß verwendeter TRPP8-Inhibitor in definierter Dosis mit einem pharmazeutisch geeigneten und physiologisch verträglichen Träger und ggf. weiteren geeigneten Wirk-, Zusatz- oder Hilfsstoffen mit definierter Inhibitordosis gemischt und zu der gewünschten Darreichungsform hergerichtet ist.

Tumorzellen exprimieren TRPP8 differenziell, wenn Normalzellen des gleichen Gewebetyps dieses nicht oder nur in sehr geringen Mengen exprimieren. Tumorzellen überexprimieren TRPP8 spezifisch bzw. differenziell, wenn TRPP8 im Vergleich zu Normalzellen des gleichen Gewebes zumindest in doppelter Menge exprimiert wird.

Zytotoxische Komponenten bzw. Gruppen sind Verbindungen, welche direkt oder indirekt Apoptose einleiten oder zumindest wachstumshemmend wirken. Solche Gruppen bzw. Verbindungen können neben Radioisotopen (z.B. 188Re, 213Bi, 99mTc, 90Y, 131J, 177Lu) insbesondere Toxine (z.B. Diphterie-Pseudomonas-Toxin) oder Zytostatika einschließlich sogenannter Prodrugs sein, welche in der Tumortherapie eingesetzt werden. Beispiele hierfür sind: Alkylantien (z.B. Mechlorethamin, Ifosfamid, Chlorambucil, Cyclophosphamid, Melphalan, Alkylsulfonate, Busulphan, Nitrosoharnstoffe, Carmustin, Lomustin, Semustin, Triazene, Dacarbazin), Antimetaboliten (z.B. Folsäure-Antagonisten, Methotrexat, Pyrimidin-Analoga, Fluoruracil, Fluordesoxyuridin, Cytarabin, Gemcitabin, Purin-Analoga, Mercaptopurin), Mitosehemmer (z.B. Vincaalkaloide, Vincristin, Vinblastin, Paclitaxal, Docetaxel, Protaxel), Epipodophyllotoxine (z.B. Etoposid, Teniposid), Antibiotika (z.B. Dactinomycin, Daunorubicin, Idarubicin, Anthracycline, Bleomycin, L-Asparaginase), Platinkomplexverbindungen (z.B. Cisplatin), Hormone und verwandte Verbindungen (z.B. Nebennierensindensteroide, Aminogluthetimid, Gestagene, Östrogene, Androgene, Antiöstrogene, Tamoxifen, Steriodanaloga, Flutamid). Bei Bindung einer solchen Verbindung mit einer an TRPP8 bindenden Substanz erfolgt die Kopplung dergestalt, daß die Affinität zu TRPP8 um nicht mehr als 90%, vorzugsweise 50%, bezogen auf die Substanz ohne zytostatische Gruppe, reduziert ist und die zytostatische Wirkung der Gruppe um nicht mehr als 90%, vorzugsweise 50%, bezogen auf die Verbindung ohne Substanz, reduziert ist.

Eine immunstimulierende Komponente ist meist ein Protein oder ein wirksamer Bestandteil hiervon, welches Zellen des Immunsystems stimuliert. Beispiele hierfür sind: Zytokine, wie M-CSF, GM-CSF, G-CSF, Interferone, wie IFN-alpha, beta, gamma, Interleukine wie IL-1 bis -16 (außer -8), human LIF, Chemokine wie Rantes, MCAF, MIP-1-alpha, -beta, NAP-1 und IL-8.

Eine Reportergruppe ist ein Atom, Molekül oder eine Verbindung, welche in Verbindung mit einem hierauf abgestellten Assay den Nachweis der Reportergruppe und der somit mit der Reportergruppe verbundenen Verbindung oder Substanz ermöglicht. Beispiele für Reportergruppen und hiermit assoziierte Detektionsmethoden sind: 32P-Labeling und Intensitätsmessung mittels Phosphoimager. Viele weitere Beispiele sind dem Durchschnittsfachmann bekannt und bedürfen nicht der detaillierten Aufzählung.

Eine an TRPP8 bindende Substanz kann eine Substanz sein, welche ein TRPP8-Protein, eine TRPP8-RNA, oder eine TRPP8-DNA bindet. Mit umfasst sind dabei bezüglich der DNA auch Inhibitoren Transkription der TRPP8 DNA und/oder Inhibtoren der die TRPP8-Genexpression regulierenden Region.

Im Rahmen der vorstehenden Definition gegenüber dem engen Wortsinn erweiterte Begriffsbestimmungen umfassen auch die bestimmten Begriffe im engen Wortsinn.

Vorbekannte Sequenzen und/oder deren Verwendung, welche unter die im Rahmen dieser Beschreibung verwendete Definitionen fallen, können durch einen Disclaimer in Patentansprüchen ausgeschlossen werden.

### Beispiele.

Im Folgenden wird die Erfindung anhand von lediglich bevorzugte Ausführungsformen darstellenden Beispielen und Abbildungen näher erläutert. Es zeigen:
- Fig. 1:: Lokalisierung der erfindungsgemäßen Trpp8 Splice Varianten relativ zu Volllängen Trpp8,
- Fig. 2:: Lokalisierung der erfindungsgemäßen Trpp8 regulatorischen RNA auf dem Chromosom 2,
- Fig. 3 bis 9:: verschiedene Hammerhead Ribozyme gegen verschiedene Trpp8 Splice Varianten sowie regulatorischer RNA, einschließlich Schnittstellen,
- Fig. 10:: Ergebnisse eines CPA,
- Fig. 11:: Ergebnisse eines Dot Blot Experimentes,
- Fig. 12:: Nachweis von 16b in einem Western Blot,
- Fig. 13:: Ergebnisse zur Kolokalisation von 16b und Trpp8,
- Fig. 14:: weitere erfindungsgemäße Sequenzen,
- Fig. 15:: TaqMan zur Überexpression von 16b in NET,
- Tab. 1:: Ergebnisse zur vergleichenden Trpp8 Expression in Prostatatumor- und -normalgewebe.

In der Abbildung 1 ist oben bekanntes Trpp8 in Volllänge dargestellt. Darunter sind die verschiedenen Splice Varianten gemäß der Erfindung dargestellt, woraus sich die Lagen der jeweiligen Exons in den erfindungsgemäßen Varianten, bezogen auf die Volllänge, entnehmen lassen.

In der Abbildung 2 ist die Lokalisierung der erfindungsgemäßen regulatorischen RNA bzw. der hierfür codierenden DNA auf dem Chromosom 2 dargestellt. Die Pfeile zeigen die Richtung der Transkription. Die erfindungsgemäße RNA entsteht durch Transkription der Exons nach links, Volllängen Trpp8 nach rechts.

### Methoden:

### 1: Mikrodissektion

Prostatatumor- und -normalgewebe aus jeweils einem Patienten wurde gefroren und in 10µm Proben geschnitten. Nach einer Färbung mit Hematoxylin wurden die Proben wieder eingefroren. Aus jedem Patienten wurden zumindest 30 Proben gewonnen. Normal- und Tumorgewebe wurde von einen Pathologen mit Hilfe eines Mikroskopes identifiziert und markiert. Die jeweiligen Bereiche wurden unter dem Mikroskop resektiert unter Verwendung einer Nadel und jeweils in 100 µl 2% Mercaptoethanol enthaltenden GTC Puffer bei -80°C eingefroren.

### 2: Poly-A+-RNA Präparation.

Die Poly-A+-RNA Präparation erfolgte unter Verwendung eines modifizierten Protokolls gemäß dem Poly-A-Tract 1000 Kit (Amersham, Freiburg, Deutschland). Gewebeproben aus 1 wurden langsam auf Eis aufgetaut, homogenisiert und mit 300µl Verdünnungspuffer, enthaltend 1% β-Mercaptoethanol, sowie biotinyliertem Oligo-dT Primer versetzt, und für 5 min. auf 70°C erhitzt. Die Proben wurden dann für 5 min. bei 20°C gehalten und anschließend bei 20000g für 10 min. zentrifugiert. Dem Überstand wurden 120µl gewaschener Streptavidin-gekoppelter paramagnetischer Partikel (SA-PMP) zugebenen und bei 20°C für 5 min. inkubiert. Die mRNA wurde dann durch magnetische Trennung isoliert. Nach drei Waschschritten mit 0,5x SSC Lösung wurde die mRNA in Nuklease-freiem Wasser eluiert, eingedampft unter Vakuum und umgehend in cDNA prozessiert.

### 3: cDNA Synthese.

Die mRNA aus 2 wurde in 10µl Nuklease-freiem Wasser gelöst. 1µl T7-dT24-(GGCCAG) Primer (100 pmol/µl) wurde zugegeben und es wurde bei 70°C für 10 min. inkubiert. Dann wurde die Probe auf Eis gelegt und 4µl 5x Erststrang Puffer (Invitrogen, Groningen, Niederlande), 2µl DTT (0,1M), 1µl dNTP's (10mM), und 14U anti-RNAse (Ambion, Huntingdon, England) zugegeben, gefolgt von einer Inkubation für 2 min. bei 37°C. Dann wurden 1µl Superscript II Reverse Transkriptase (Invitrogen) zugegeben, gefolgt von einer Inkubation für 1 h bei 37°C.

### 4: Zweitstrangsynthese und DNA Reinigung.

Sofort nach der Synthese des ersten Stranges nach 3 wurden 91µl Wasser, 30µl 5x Zweitstrang Puffer, 3µl dNTP's (10mM), 10U E. coli DNA-ligase, 40U DNA Polymerase I und 2U RNAse H (alle von Invitrogen) zugegeben und die Mischung wurde für 2 h bei 16°C inkubiert. Dann wurden 10U T4 DNA Polymerase (Invitrogen) zugegeben und weitere 5 min. inkubiert. Die Reaktion wurde durch Zugabe von 10µl 0,5mM EDTA gestoppt. Die Reinigung der DNA erfolgte gemäß den Vorschriften des GFX PCR DNA and Gel Band Purification Kits (Amersham Biosciences, Buckinghampshire, England). Gereinigte DNA wurde unter Vakuum eingedampft und bei -20°C gelagert.

### 5: In vitro Transkription und cRNA Reinigung.

Die in vitro Transskription wurde gemäß dem Herstellerprotokoll von Ambion durchgeführt. Das DNA Pellet wurde in 8µl Wasser gelöst und 7,5µl dNTP's (75mM), 2µl 10x reaction buffer (Ambion), 2µl 10 T7 Enzymmix (Ambion) und 14U anti-RNAse (Ambion) wurden zugegeben, gefolgt von einer Inkubation von 6 h bei 37°C. Die Reinigung der erhaltenen cRNA erfolgte gemäß dem Herstellerprotokoll zum Rneasy Mini Kit (Qiagen, Hilden, Deutschland). Nach Elution von der Säule wurde die cRNA eingedampft unter Vakuum und auf -80°C eingefroren.

### 6: Zweite in vitro Transskriptionsrunde.

Die zweite Amplifikationsrunde wurde mit nur geringen Abweichungen von der ersten Runde durchgeführt. Die Synthese des ersten Stranges erfolgte mit random hexamer primer (250ng/µl). Nach Inkubation über 60 min. bei 37 °C wurde das cRNA-cDNA Hybrid bei 37 °C für 20 min. mit 2U RNase H inkubiert, gefolgt von einem 2-minütigen Inaktivierungsschritt bei 95°C.

### 7: Quantitative PCR und Auswertung.

Die Synthese des ersten Stranges erfolgte mit der cRNA aus 6. 1ng cDNA wurden für die Amplifikation eingesetzt mit 2,5µl 10x SYBR®Green PCR Puffer, 3µl Magnesiumchlorid (25mM), 2µl dNTP's (mit dUTP; 12,5 mM) und 0,625U Ampli Taq Gold in einem Reaktionsvolumen von 25µl. Die Reaktion wurde in einem GeneAmp 5700 Sequence Detection System (Applied Biosystems, Weiterstadt, Deutschland) durchgeführt. Die Bedingungen waren: 2 min. 50°C, 10 min. 95°C, 15 s 95°C, 1 min. 60°C, die letzten beiden Phasen in 40 Zyklen. Für 4a_4b wurden die Vorwärts- bzw. Rückwärtsprimer gemäß Seq.-ID 9 bzw. 10 verwendet, für 6b 11 bzw. 12, für 16b 13 bzw. 14, für 20b 15 bzw. 16, für avant25 17 bzw. 18, für avant13 19 bzw. 20, und für beta-Aktin 21 bzw. 22. Die Auswertung erfolgte nach der ΔΔCt Methode nach Herstellervorschrift. Der CT Wert des Kontrollgens beta-Aktin wurde bei einer Grenze von 0,1 gemessen. Zur Normalisierung wurde der CT Wert des beta-Aktin vom CT Wert des untersuchten Gens abgezogen. Dieser normalisierte CT Wert wurde im Falle der Tumorgewebe auf die Normalgewebe bezogen bzw. normalisiert, wodurch der ΔΔCt erhalten wird. Wird dieser Wert als Potenz zur Basis 2 eingesetzt, so wird eine relative Größe der Über- oder Unterexpression in Tumorgewebe gegenüber dem Normalgewebe des gleichen Patienten erhalten.

### Beispiel 1: Vergleich der Expression erfindungsgemäßer Trpp8 Sequenzen in Tumorgewebe gegenüber Normalgewebe der jeweiligen Patienten.

Für die Proben jedes Patienten wurden quantitative PCR Versuche gemäß dem Teil "Methoden" durchgeführt. Überexpression wurde für 4-fache und höher Werte im Tumorgewebe und Unterexpression für 0,25-fache und niedriger Werte definiert. Die Ergebnisse sind in der Tabelle 1 zusammengefaßt. Zum Vergleich wurde die Expression von Volllängen Trpp8 aus demselben Patientenkollektiv unter identischen Bedingungen analysiert.

**Tabelle 1**

| Gen | Anzahl Patienten mit Überexpr. | Anzahl Patienten mit Unterexpr. |
|---|---|---|
| Trpp8 | 23 aus 42 (55%) | 1 aus 42 (2%) |
| | | |
| 6b | 7 aus 14 (50%) | 1 aus 14 (7%) |
| 16b | 28 aus 43 (65%) | 0 aus 43 (0%) |
| 20b | 28 aus 42 (67%) | 2 aus 42 (5%) |
| avant25 | 9 aus 15 (60%) | 0 |
| avant13 | 7 aus 14 (50%) | 0 |
| 4a_4b | 8 aus 14 (58%) | 3 aus 14 (21%) |
| reg. RNA | 12 aus 15 (80%) | 1 aus 15 (7%) |

Man erkennt, daß die erfindungsgemäßen Gene bzw. Splice Varianten oder regulatorische RNA mit hoher, teilweise sehr hoher Spezifität als Marker für Tumorgewebe fungieren. Entsprechend hoch ist die Anwendungsbreite der jeweiligen Inhibierung für therapeutische Zwecke mittels der beschriebenen bindenden Substanzen.

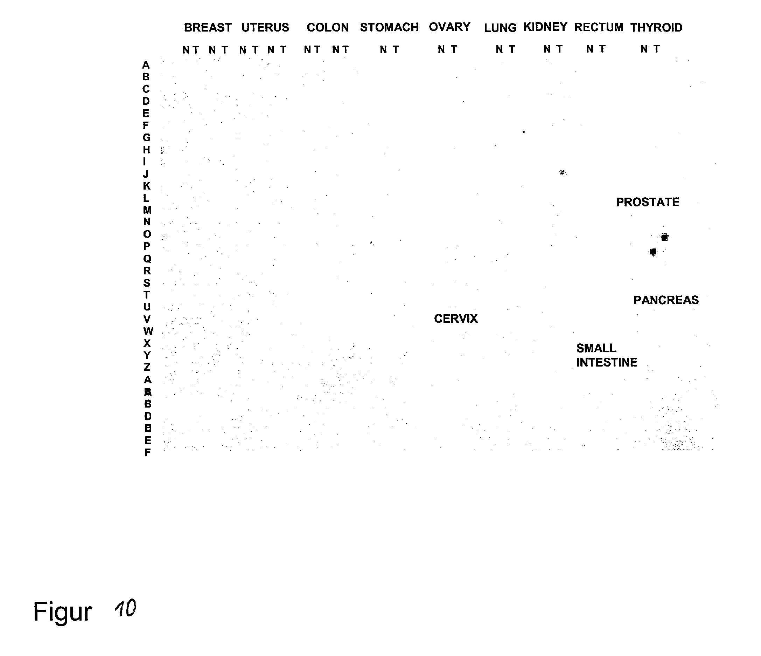

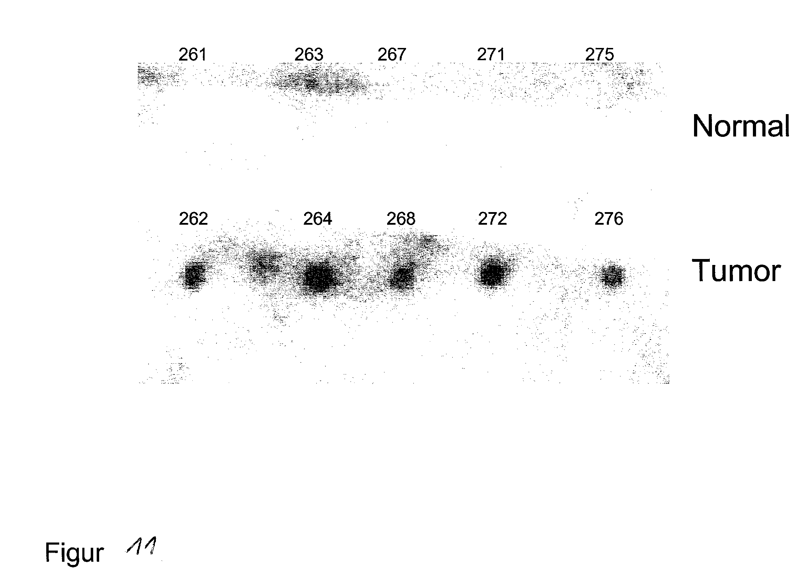

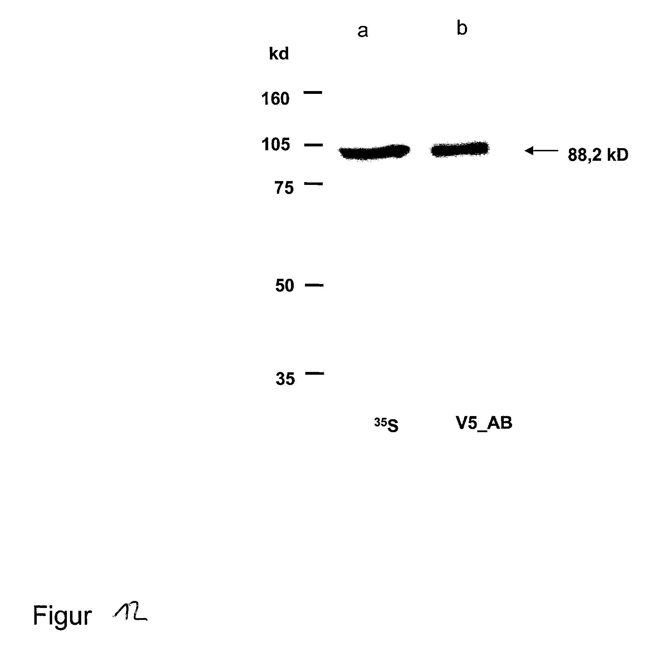

In der Figur 10 sind Ergebnisse eines Cancer Profiling Array (CPA) dargestellt. Eine Splicevariante 16b spezifisches cDNA Fragment wurde mit 32P markiert und auf ein CPA (Invitrogen) hybridisiert, welcher 241 cDNAs von Tumorund korrespondierendem Normalgewebe von unterschiedlichen Patienten enthält. "N" steht dabei für Normalgewebe und "T" für Tumorgewebe. Man erkennt Expression von Trpp8 in Prostata- sowie Nierentumorgewebe, wobei die Überexpression im Falle des Prostatagewebes prominent ist.

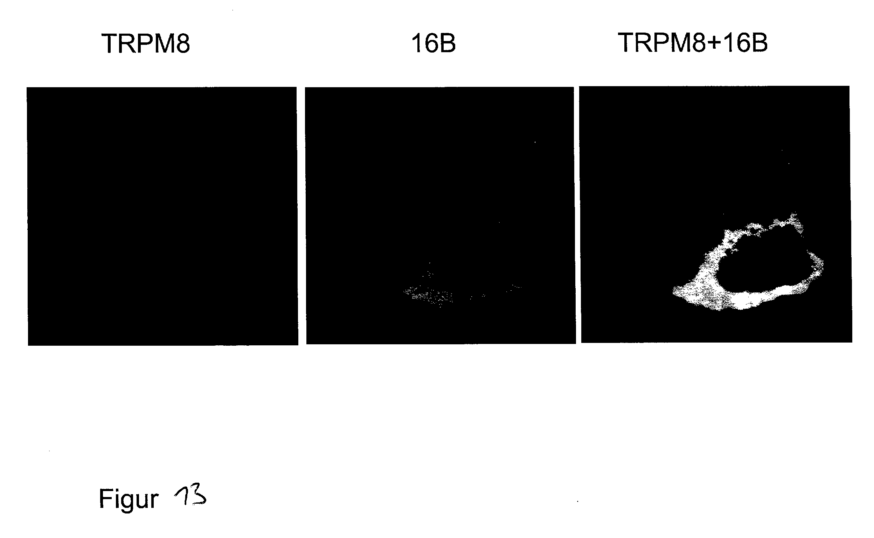

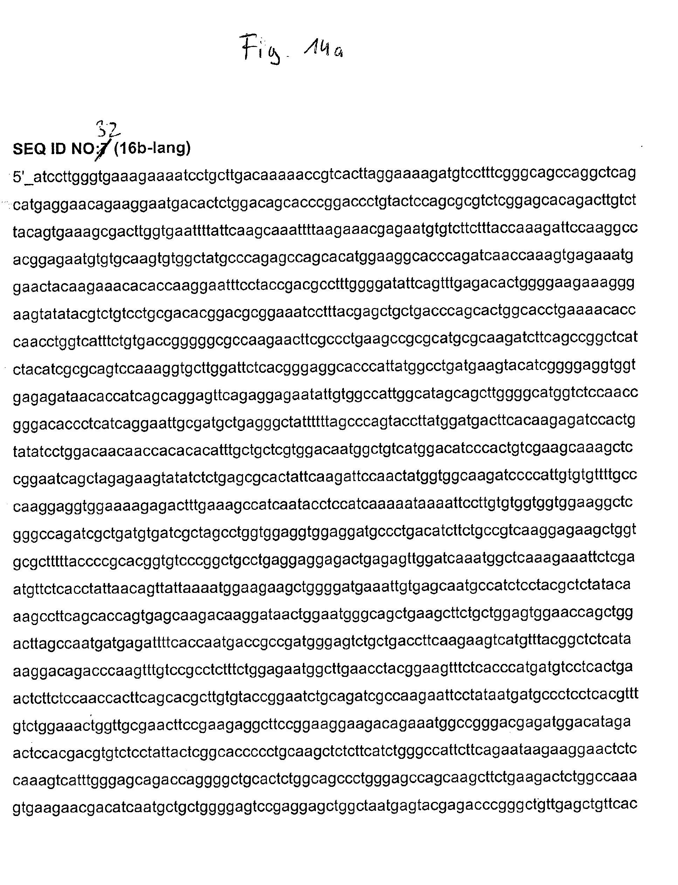

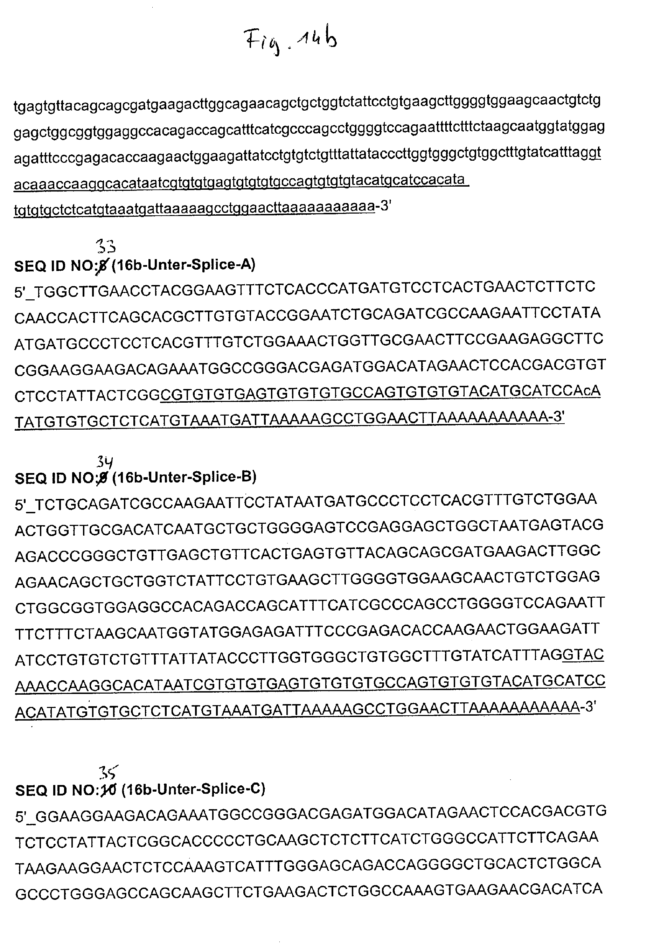

In der Figur 11 sind Ergebnisse eines Dot Blot Experimentes dargestellt, und zwar von 5 verschiedenen humanen Prostata Tumor- und korrespondierenden Normalgeweben. Die Durchführung erfolgte entsprechend einem Northern Blot, jedoch ohne vorherige Auftrennung der Proben in einem Agarosegel. Die Analyse erfolgte anhand von amplifizierter mRNA (cRNA) aus 6 Patienten (Tumor/Normal). Die cRNA aus der zweiten Amplifikationsrunde wurde auf eine Membran gespottet, fixiert und anschließend mit einer Splicevariante 16b spezifischen Sonde (32P markiert) hybridisiert. Man erkennt, dass die Splicevariante 16b ausschließlich in den Protata Tumoren exprimiert wird.

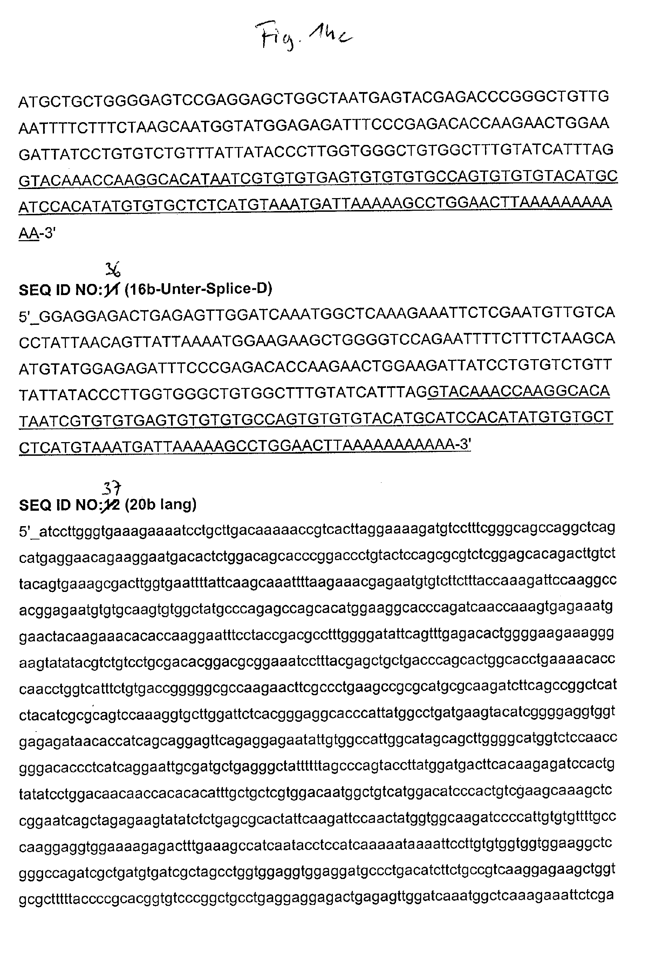

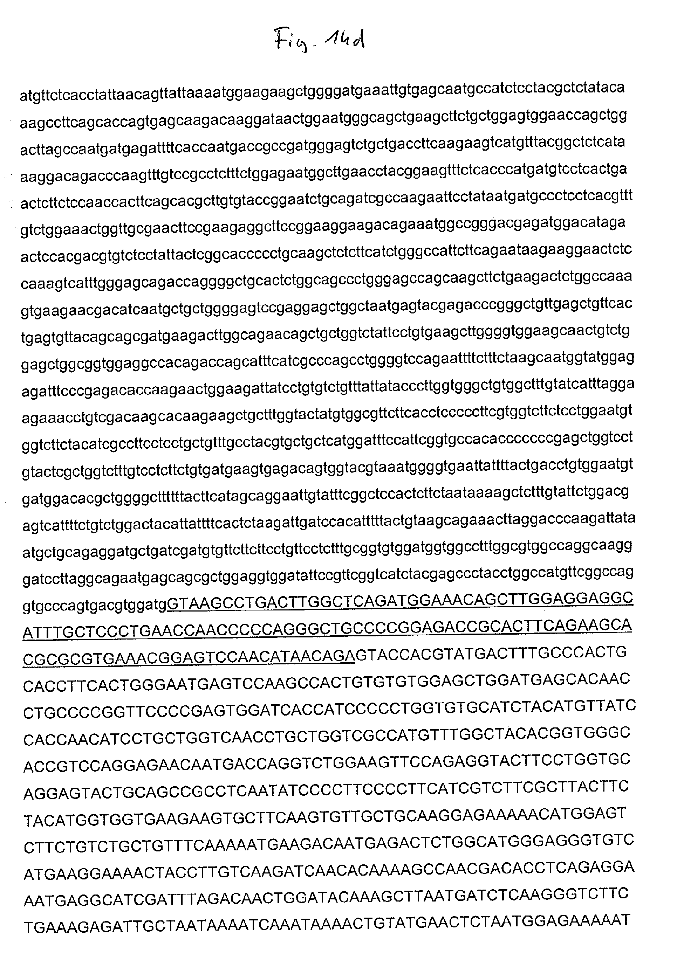

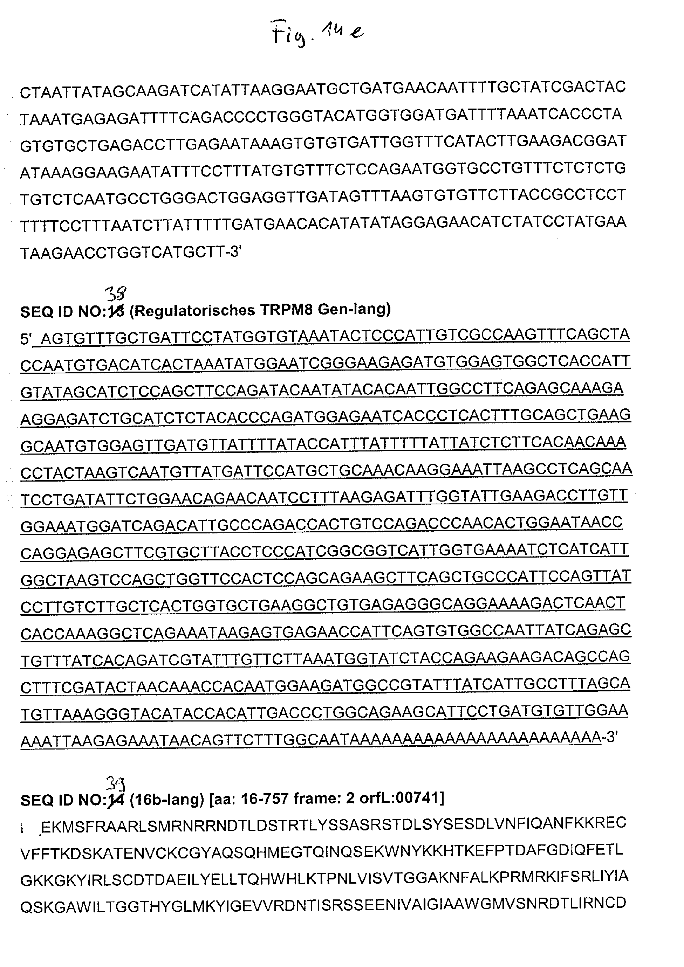

In der Figur 12 ist ein Nachweis des Proteins der Variante 16b in einem Western Blot dargestellt. Hierbei wurde ein Konstrukt erzeugt, enthaltend die verlängerten Variante 16b, kloniert in den Vektor pcDNA3.1/V5-His-TOPO (Invitrogen). Die Expresssion der Splicevariante 16b erfolgte durch in vitro Translation in einem Retikulozytenlysat (Promega). Der Nachweis erfolgte durch Einbau von 35S-markiertem Methionin (a) bzw. in Abwesenheit von 35S-markiertem Methionin. Im Falle b wurde der Western Blot mit einem Anti-V5-Antikörper gegen den V5-Tag der klonierten Variante 16b nachgewiesen. Die erhaltenen Produkte entsprechen in beiden Fällen a und b der exakten Größe der 16b Variante einschließlich des V5-Tags (88,2 kD).

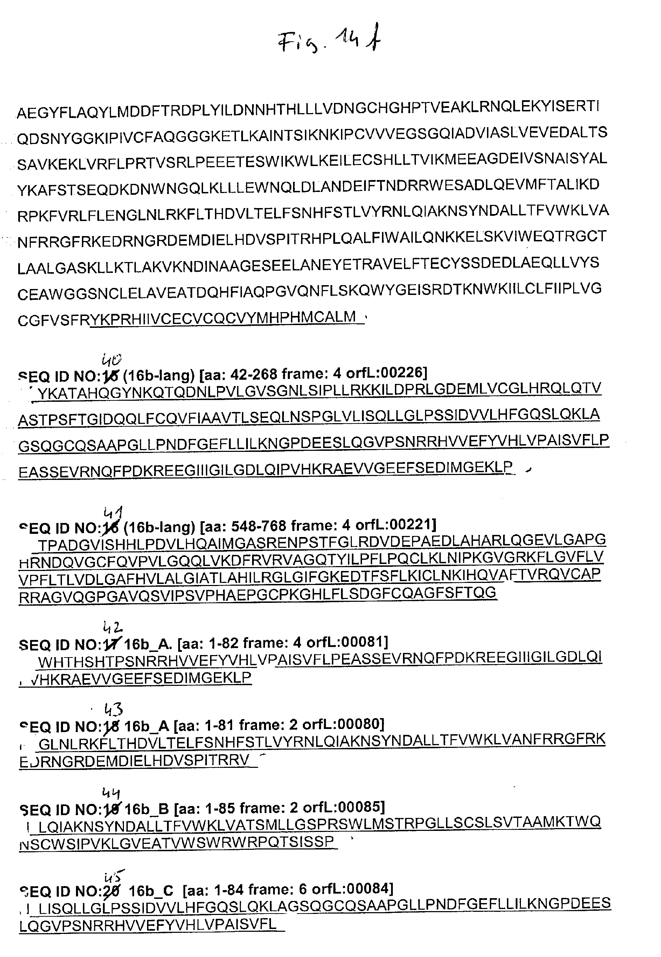

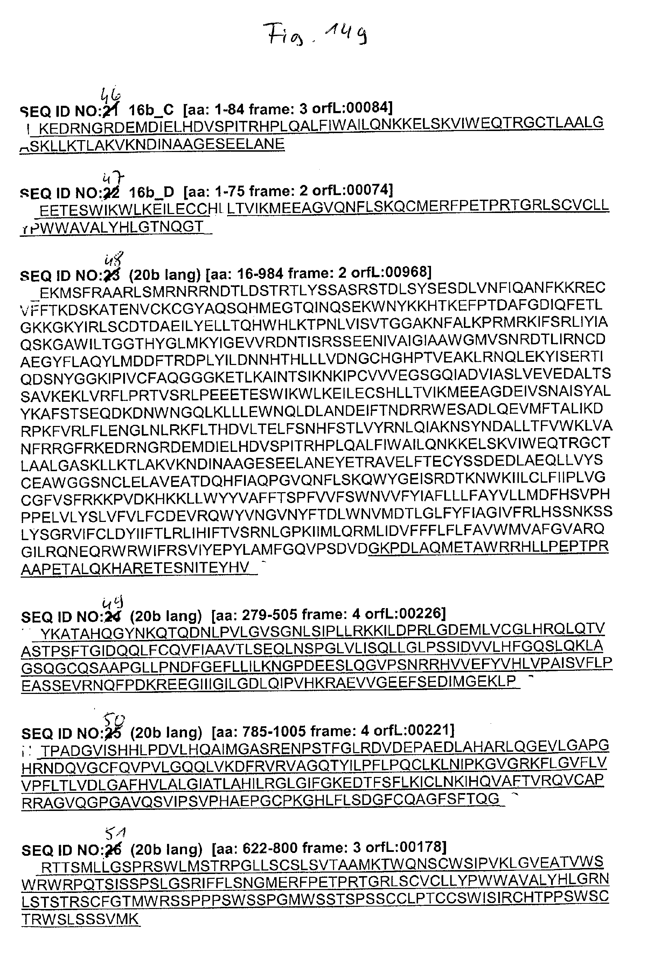

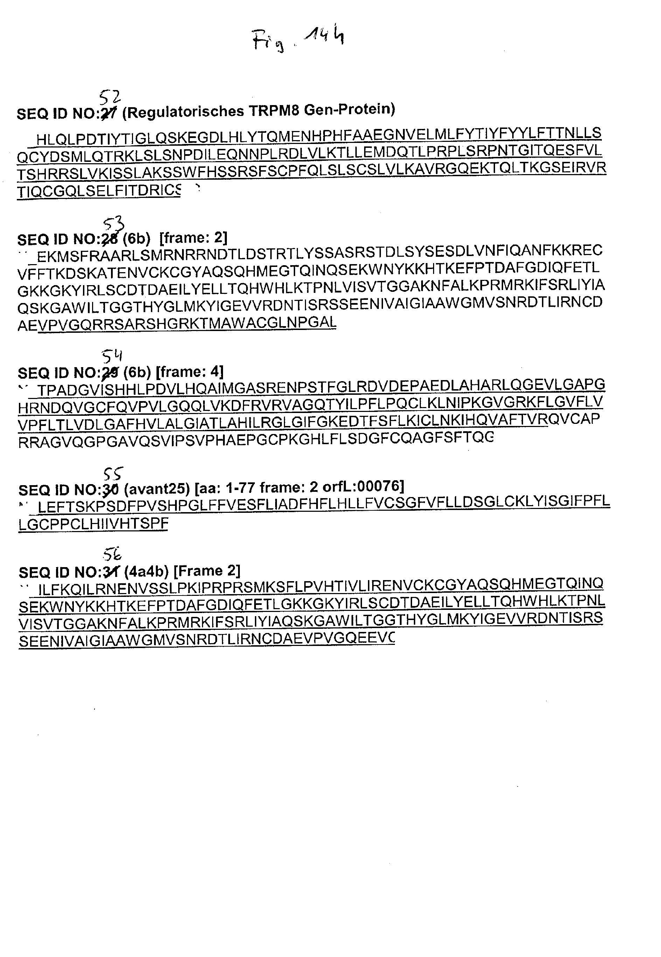

Figur 13 zeigt Ergebnisse zur Kolokalisation von Trpp8 und der Splicevariante 16b. Trpp8 Volllänge cDNA wurde in den Expressionsvektor pcDNA3.1-V5his subkloniert und mittels Lipofektion in HEK293 Zellen transfiziert. Stabile Klone wurden durch Selektion in Gegenwart von 1 mg Neomycin pro ml DMEM Medium mit 10% FKS generiert. Die Expression wurde durch Immuno-Blot Analyse mit anti-V5 spezifischen Antikörpern nachgewiesen. Die cDNA der Trpp8 Isoform 16b wurde in den Expressionsvektor pcDNA6myc-his subkloniert und mittels Lipofektion transient in HEK293 Zellen, die Trpp8 stabil exprimieren, transfiziert. Für die Lokalisation der Trpp8 Expressionskonstrukte wurde eine immunzytochemische Färbung durchgeführt. Dazu wurden Zellen, die auf Glasdeckgläschen kultiviert wurden, mit PBS gewaschen und mit 4% para-Formaldehyd fixiert. Nach erneutem PBS Waschen wurden die Zellen mit 0,2% Triton X-100 in PBS permeabilisiert und nach Absättigung unspezifischer Bindungen mit 0,2% Fisch-Gelantine in PBS mit Maus-Antikörpern gegen myc-Epitope und Kaninchen-Antikörpern gegen V5-Epitope inkubiert. Anschließend wurden die Zellen erneut gewaschen und mit fluoreszenzmarkierten Antikörpern gerichtet gegen Maus (488 nm) und Kaninchen (594 nm) inkubiert. Nach Färbung der Zellkerne mit 2 µg/ml DAPI in PBS wurden die Zellen auf Objektträgern durch Einbettung fixiert. Die Visualisierung der Zelllokalisation der Trpp8 Isoform 16b wurde mit einem konfokalen Lasermikroskop durchgeführt. Die sequenziell aufgenommenen Bilder wurden abschließend überlagert. In A dargestellt sind Trpp8 exprimierende Zellen (V5-markiert), B zeigt 16b koexprimierende Zellen (mycmarkiert) und C die Überlagerung der Bilder A und B. Man erkennt deutlich signifikante Kolokalisation.

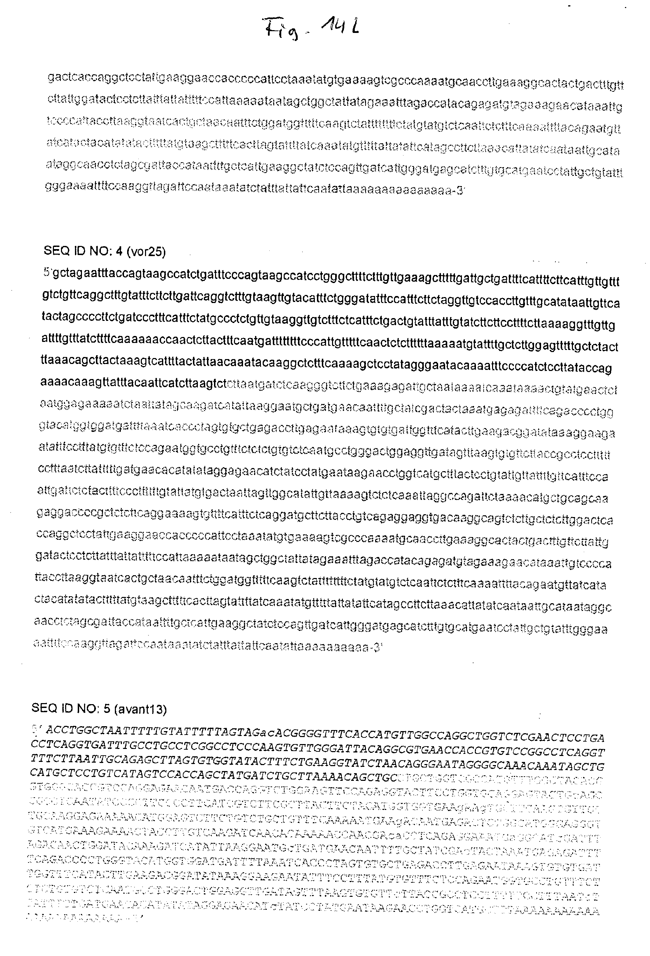

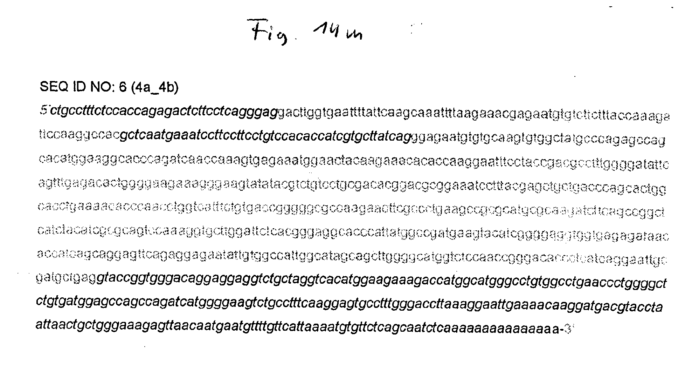

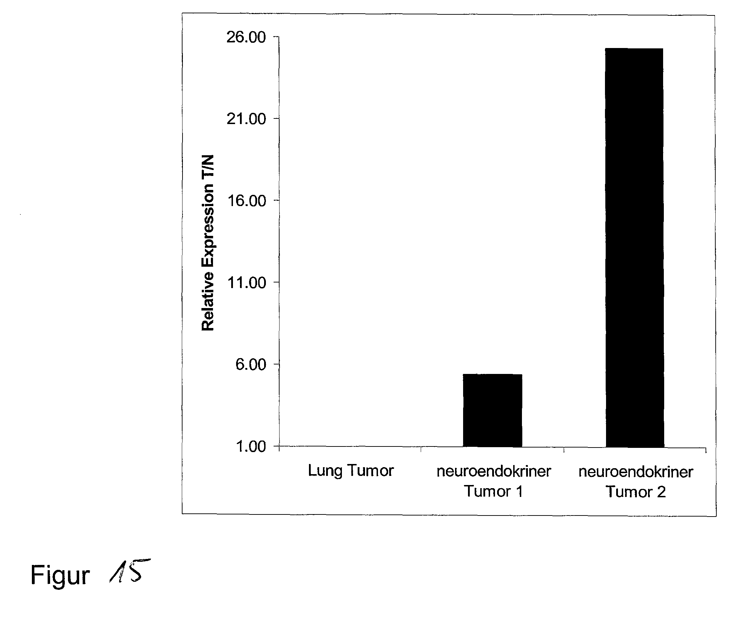

In Untersuchungen mittels quantitativer PCR (TaqMan) wurde festgestellt, dass Trpp8 sowie Splicevarianten auch in neuroendokrinen Tumoren (NET) spezifisch überexprimiert wird. In der Figur 15 sind Ergebnisse für die Splicevariante 16b dargestellt und man erkennt signifikante Überexpression. Als Kontrolle wurde ein Lungentumor verwendet, der zur Gruppe der Adenokarzinome gehört, für welche keine Überexpression von Trpp8 detektiert werden kann. Die Expression von Trpp8 und allen Splicevarianten ist (im Gegensatz zum Adenokarzinom) in der Gruppe der kleinzelligen Lungenkarzininome immer erhöht gegenüber dem Normalgewebe. NETs leiten sich immer aus Nervenzellen ab und können sich aus verschiedenen Geweben entwickeln. Die Überexpression von Trpp8 und den Splicevarianten tritt generell in allen NETs auf, unabhängig davon, in welchem Gewebe sie sich ansiedeln.

### Beispiel 2: Herstellung von Antikörpern

Durch Immunisierung mit einem Trpp8 Antigen können in fachüblicher Weise polyklonale Antikörper und nach Selektion von Hybridomzellen monoklonale Antikörper hergestellt werden, welche sowohl im therapeutsichen als auch im diagnostischen Bereich Anwendung finden können. Als Immunogene kommen beispielsweise in Frage: i) Prostatatumorzelllinien, die Trpp8 Splice Varianten endogen exprimieren, beispielsweise LNCaP, ii) mit Trpp8 Splice Varianten transient bzw. stabil transfizierte Prostatatumorzelllinien, iii) mit Trpp8 Splice Varianten transient bzw. stabil transfizierte Zelllinien wie NIH3T3, COS7, oder HEK293, oder vi) cDNA der Trpp8 Splice Varianten zur Immunisierung nicht-menschlicher Säugetiere. Es ist auch die Immunisierung mittels Peptiden möglich, für welche eine Splicevariante oder ein Teil hiervon codiert. Schließlich ist eine Immunisierung mit rekombinant in Insektenzellen hergestellten Trpp8 Splice Varianten möglich.

### Beispiel 3: Antisense Oligonukleotide

Ein Beispiel eines antisense Oligonukleotides gegen 6b ist tcc tgt ccc acc ggt acc tc. Ein Beispiel eines antisense Oligonukleotides gegen 16b ist tgt gcc ttg gtt tgt acc ta. Ein Beispiel eines antisense Oligonukleotides gegen 20b ist cca agt cag gct tac cat cc. Ein Beispiel eines antisense Oligonukleotides gegen avant13 ist gcg acc agc agg cag ctg tt. Ein Beispiel eines antisense Oligonukleotides gegen 4a_4b ist gga ttt cat tga gcg tgg cc. Ein Beispiel eines antisense Oligonukleotides gegen avant25 ist gaa atc aga tgg ctt act gg.

### Beispiel 4: Ribozyme gegen Trpp8 Splice Varianten sowie regulatorische RNA.

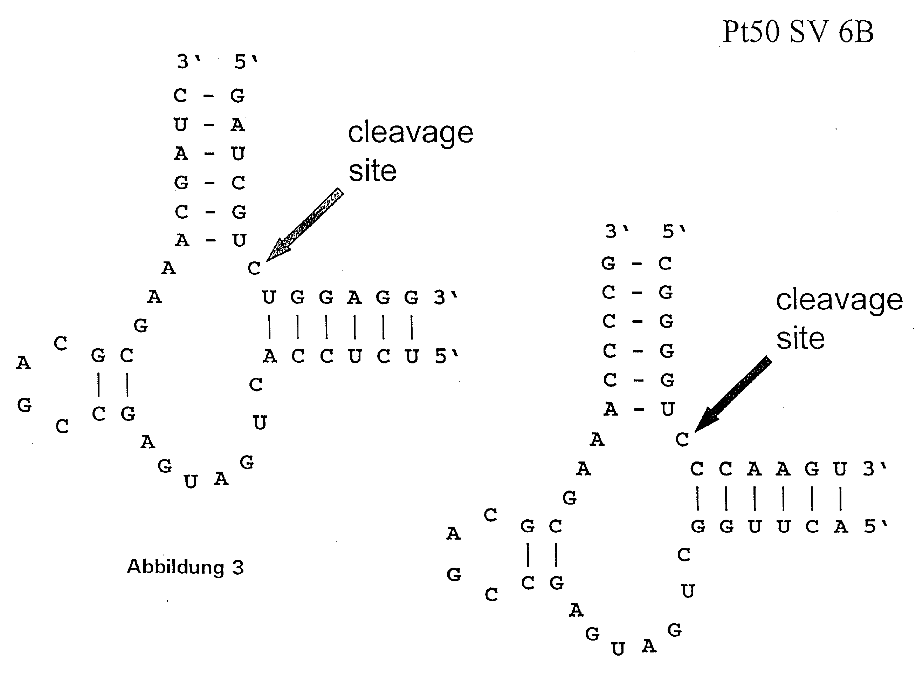

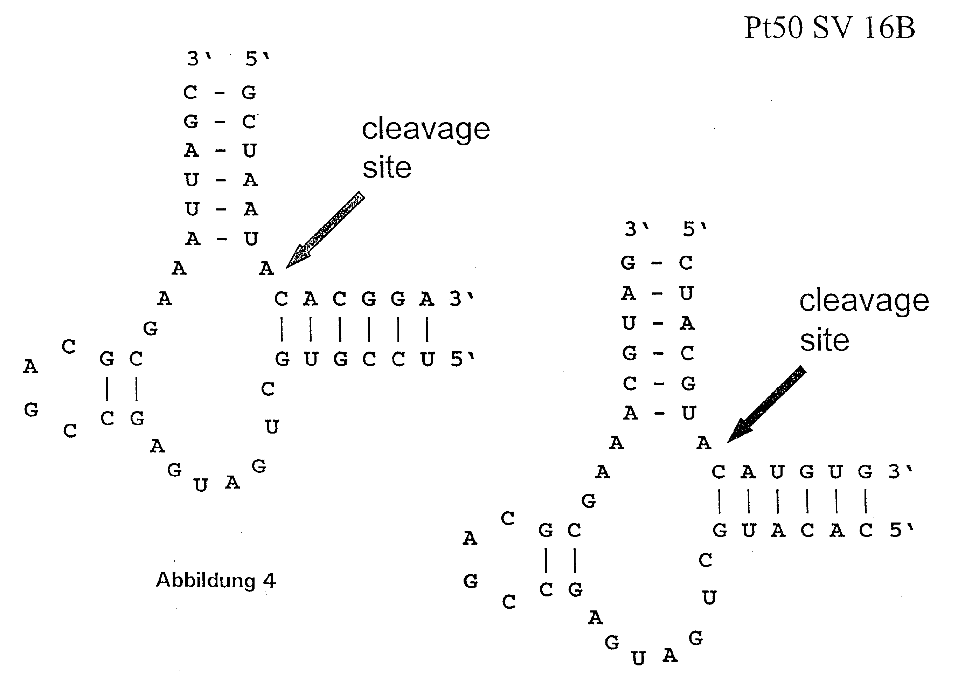

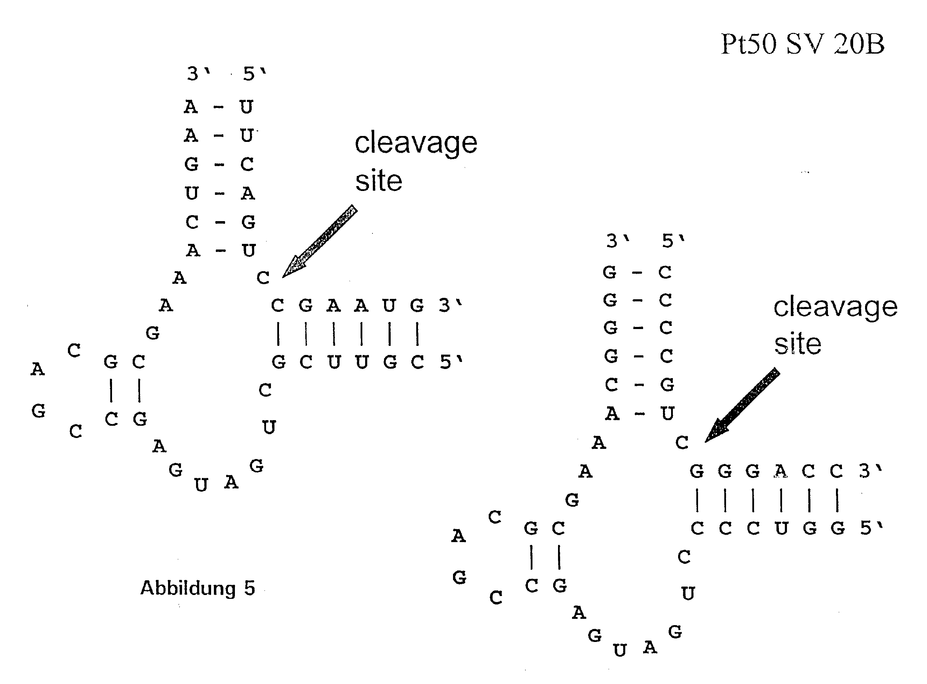

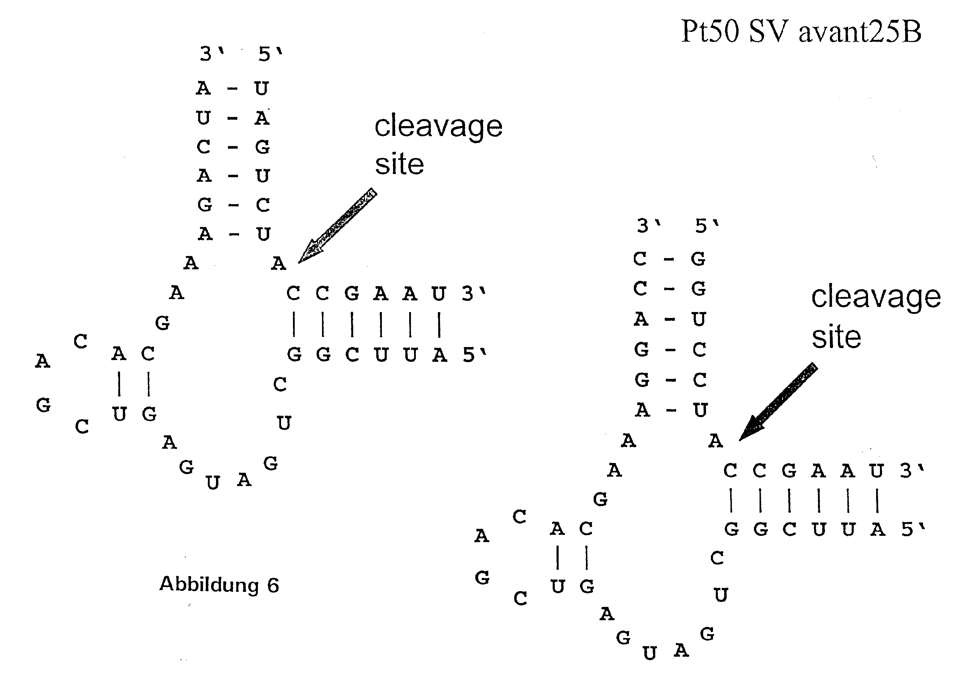

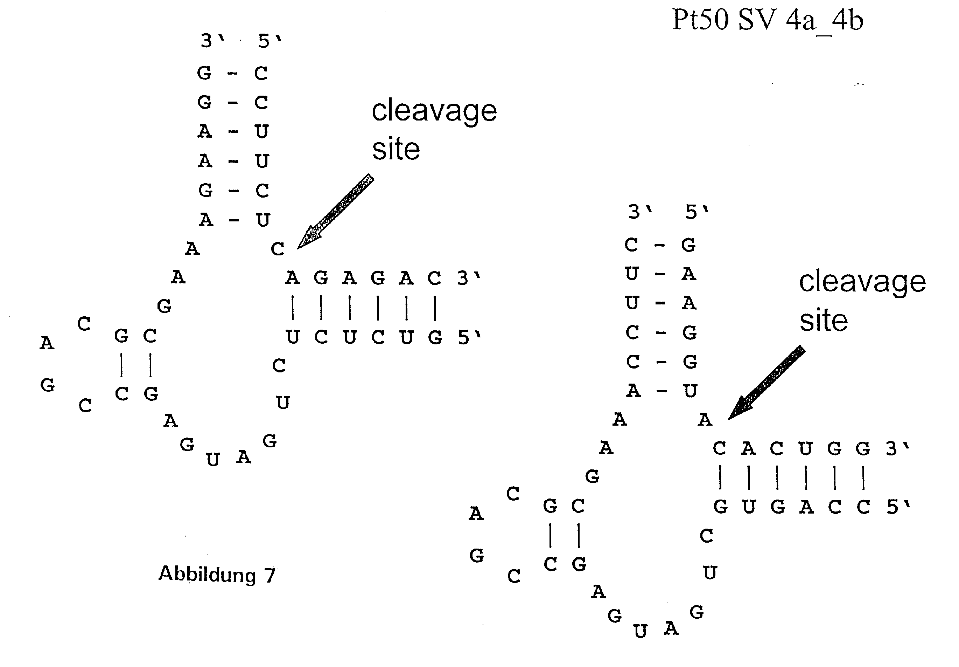

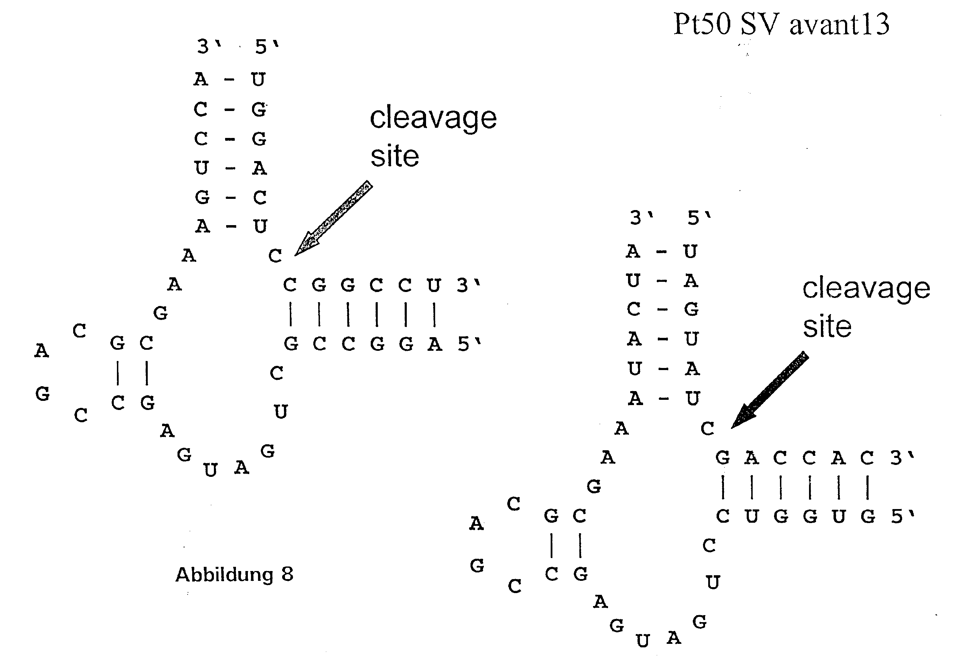

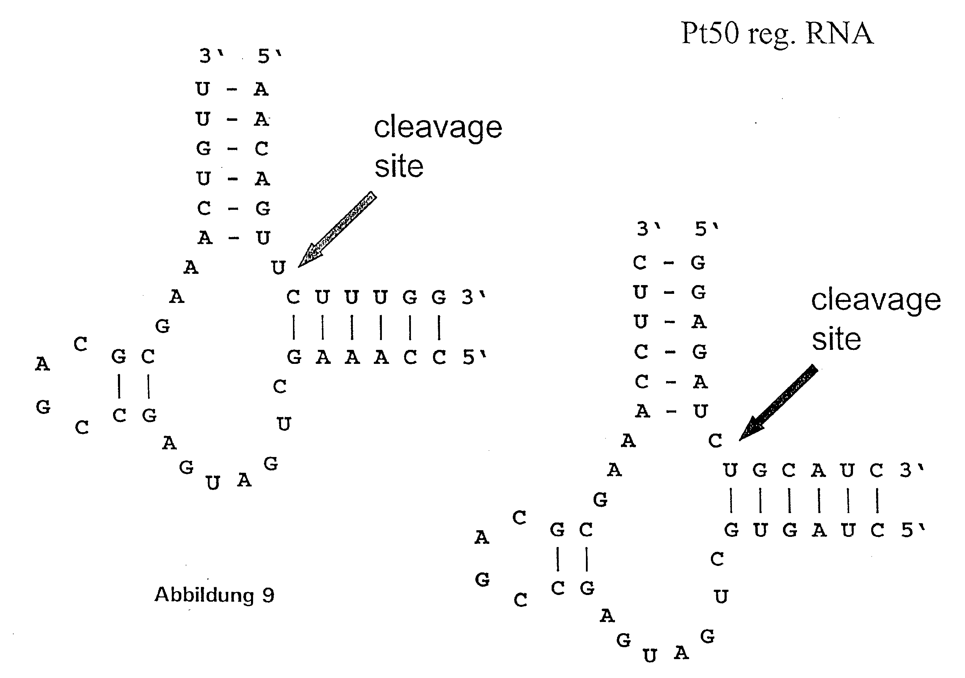

In den Abbildungen 3 bis 9 sind verschiedene Hammerhead Ribozyme dargestellt welche die in den Abbildungen angegebenen Trpp8 Splice Varianten bzw. die regulatorische RNA an den angegebenen Stellen schneiden.

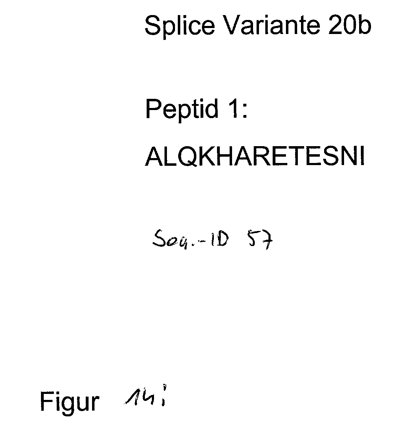

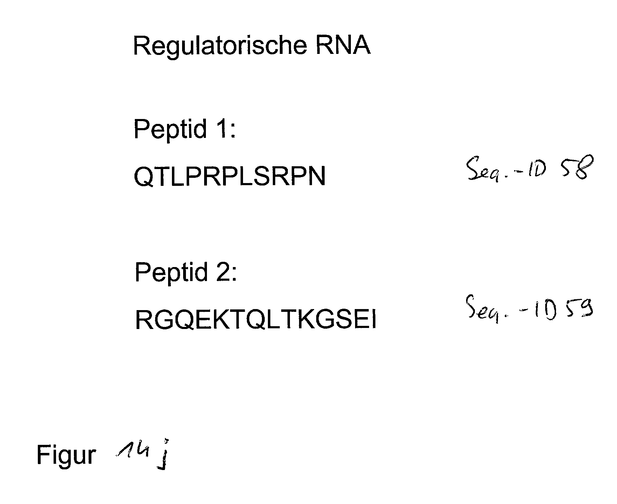

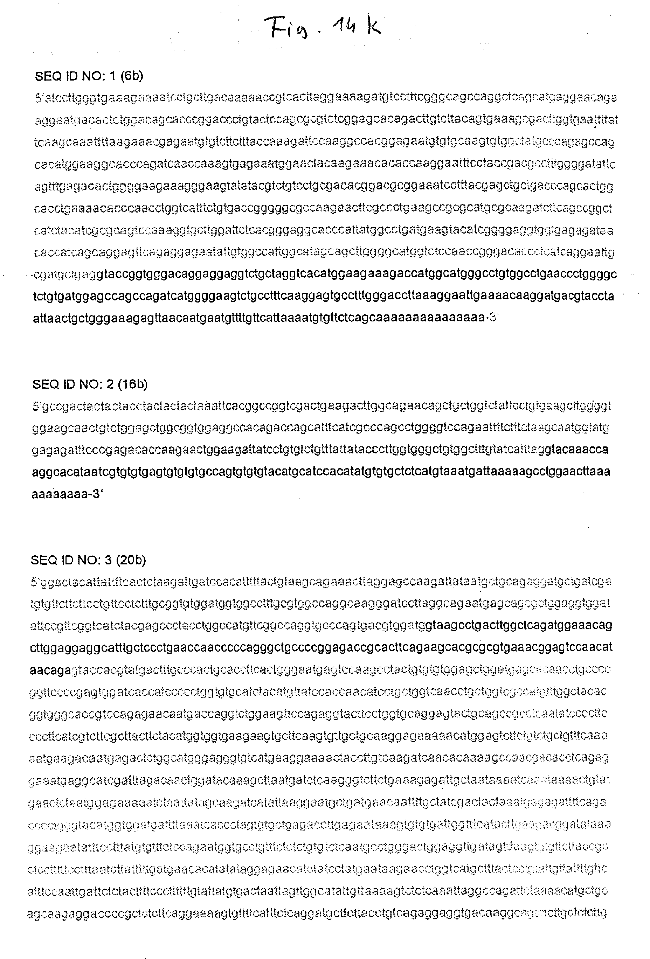

Erfindungsgemäße Sequenzen sind in der Figur 14 sowie in den Sequenzprotokollen angegeben ("vor" = "avant").

## Patentansprüche

1. Nukleinsäure codierend für eine Trpp8 Splice Variante oder regulatorische RNA enthaltend eine Nukleinsäuresequenz gemäß einer der Sequenzen Seq.-ID 1 bis 7 oder 23 bis 38, oder bestehend hieraus, optional isoliert und/oder Trpp8-funktional.

2. Trpp8 Peptid oder Protein enthaltend eine Aminosäurensequenz codiert durch eine der Nukleinsäuresequenzen Seq.-ID 1 bis 7 oder 23 bis 38, oder gemäß Seq.-ID 8 oder Seq.-ID 39 bis 59, optional isoliert und/oder Trpp8-funktional.

3. Verwendung einer für Trpp8 codierenden Nukleinsäure und/oder eines Trpp8 Peptids oder Proteins, insbesondere nach Anspruch 1 oder 2, zur Detektion von Krebs oder zur Detektion eines Risikos der Erkrankung an Krebs, insbesondere Prostatakrebs, Nierenkrebs oder neuroendokrinen Tumoren (NET), wobei eine Gewebeprobe, beispielsweise eine Prostata-Gewebeprobe oder Nierengewebeprobe, auf Übertranskription von Trpp8 RNA oder auf Überexpression eines Trpp8 Proteins untersucht wird.

4. Verwendung nach Anspruch 3, wobei eine an für Trpp8 codierende Nukleinsäure oder eine an Trpp8 Protein oder Peptid bindende Detektorsubstanz, vorzugsweise enthaltend eine Reportergruppe, verwendet wird, wobei Bindung besagter Nukleinsäure und/oder besagten Proteins oder Peptids an die Detektorsubstanz halbquantitativ oder quantitativ detektiert wird.

5. Verwendung einer Trpp8 DNA, insbesondere mit regulatorischen Eigenschaften, einer Trpp8 RNA oder eines Trpp8 Proteins oder Peptids, insbesondere nach Anspruch 1 oder 2, zum Screenen nach daran bindenden Substanzen, insbesondere prospektiven Wirkstoffen zur Inhibierung von besagter DNA (insbesondere deren Transkription), RNA oder besagtem Protein oder Peptid, oder prospektiven Detektorsubstanzen, wobei eine prospektive Substanz oder eine Mischung solcher prospektiver Substanzen mit besagter DNA, RNA oder besagtem Protein oder Peptid kontaktiert wird, wobei mit einem Bindungsassay Bindungsereignisse festgestellt werden, und wobei eine bindende prospektive Substanz, ggf. nach Dekonvolutierung, selektiert wird.

6. Verwendung einer Trpp8 inhibierenden oder daran bindenden Substanz zur Herstellung einer pharmazeutischen Zusammensetzung zur Behandlung von Krebs, insbesondere Prostatakrebs, Nierenkrebs oder NET.

7. Verwendung nach Anspruch 6, wobei die Substanz ein Antikörper ist, welcher durch Immunisierung eines nicht-menschlichen Säugetiers mit einer Trpp8 cDNA, einem Trpp8 Peptid oder Protein, insbesondere gemäß Anspruch 2, erhältlich ist oder ein Phage-Display Antikörper ist.

8. Verwendung nach Anspruch 7, wobei die Substanz eine Mimikryverbindung eines Antikörpers gegen ein Trpp8 Peptid oder Protein, insbes. gemäß Anspruch 2, ist.

9. Verwendung nach Anspruch 6, wobei die Substanz ein Aptamer, eine antisense RNA, eine inhibitorische RNA, eine RNAi oder ein Ribozym ist.

10. Verwendung nach einem der Ansprüche 6 bis 9, wobei die Substanz zusätzlich eine zytotoxische und/oder immunstimulierende Komponente trägt.

11. Verwendung nach einem der Ansprüche 6 bis 10, wobei die pharmazeutische Zusammensetzung zur lokalen Applikation in Tumorzellen enthaltendem Gewebe hergerichtet ist.

12. Verfahren zur Diagnose einer Krebserkrankung, wobei eine Detektorsubstanz nach Anspruch 4 in der Ausführungsform mit einer Reportergruppe in zu untersuchendes Gewebe appliziert wird, wobei das zu untersuchende Gewebe dann einer Detektionsverfahrenstufe unterworfen wird, welche sensitiv für die Reportergruppe ist, und wobei im Fall der Detektion eines definierten Mindestwertes der Reportergruppe im Gewebe das Gewebe als Tumorzellen enthaltend qualifiziert wird.

13. Verfahren zur Behandlung einer Krebs-Erkrankung bzw. Behandlungsplan, wobei eine pharmazeutische Zusammensetzung nach einem der Ansprüche 6 bis 11 in einer physiologisch wirksamen Dosis einem Patienten dargereicht wird.

14. Verfahren zur Behandlung einer Krebs-Erkrankung bzw. Behandlungsplan, wobei zunächst eine Detektorsubstanz nach Anspruch 4 in der Ausführungsform mit einer Reportergruppe in zu untersuchendes Gewebe appliziert wird, wobei das zu untersuchende Gewebe dann einer Detektionsverfahrenstufe unterworfen wird, welche sensitiv für die Reportergruppe ist, und wobei im Fall der Detektion eines definierten Mindestwertes der Reportergruppe im Gewebe das Gewebe als thermosensitive Tumorzellen enthaltend qualifiziert wird, und wobei dann eine Krebsthermotherapie, beispielsweise transurethrale Mikrowellen-Hyperthermie oder transrektale Ultraschall-Hyperthermie, durchgeführt wird.

15. Verfahren nach Anspruch 14, wobei vor, zugleich oder nach der Krebsthermotherapie eine pharmazeutische Zusammensetzung nach einem der Ansprüche 6 bis 11 dargereicht wird.


SEQUENCE LISTING

<110> metaGen Pharmaceuticals GmbH

<120> Trp-p8 Splice Varianten und regulatorische RNA

<130> MET/DE/0203

<160> 31

<170> PatentIn version 3.1

<210> 1
<211> 1000
<212> DNA
<213> Homo sapiens

<400> 1
atccttgggt gaaagaaaat cctgcttgac aaaaaccgtc acttaggaaa agatgtcctt 60

tcgggcagcc aggctcagca tgaggaacag aaggaatgac actctggaca gcacccggac 120

cctgtactcc agcgcgtctc ggagcacaga cttgtcttac agtgaaagcg acttggtgaa 180

ttttattcaa gcaaatttta agaaacgaga atgtgtcttc tttaccaaag attccaaggc 240

cacggagaat gtgtgcaagt gtggctatgc ccagagccag cacatggaag gcacccagat 300

caaccaaagt gagaaatgga actacaagaa acacaccaag gaatttccta ccgacgcctt 360

tggggatatt cagtttgaga cactggggaa gaaagggaag tatatacgtc tgtcctgcga 420

cacggacgcg gaaatccttt acgagctgct gacccagcac tggcacctga aaacacccaa 480

cctggtcatt tctgtgaccg gggcgccaag gaacttcgcc ctgaagccgc gcatgcgcaa 540

gatcttcagc cggctcatct acatcgcgca gtccaaaggt gcttggattc tcacgggagg 600

cacccattat ggcctgatga agtacatcgg ggaggtggtg agagataaca ccatcagcag 660

gagttcagag gagaatattg tggccattgg catagcagct tggggcatgg tctccaaccg 720

ggacaccctc atcaggaatt gcgatgctga ggtaccggtg ggacaggagg aggtctgcta 780

ggtcacatgg aagaaagacc atggcatggg cctgtggcct gaaccctggg gctctgtgat 840

ggagccagcc agatcatggg gaagtctgcc tttcaaggag tgcctttggg accttaaagg 900

aattgaaaac aaggatgacg tacctaatta actgctggga aagagttaac aatgaatgtt 960

ttgttcatta aaatgtgttc tcagcaaaaa aaaaaaaaaa 1000

<210> 2
<211> 391

<212>  DNA
<213>  Homo sapiens

<400>  2
gccgactact actacctact actactaaat tcacggccgg tcgactgaag acttggcaga      60

acagctgctg gtctattcct gtgaagcttg gggtggaagc aactgtctgg agctggcggt     120

ggaggccaca gaccagcatt tcatcgccca gcctggggtc cagaattttc tttctaagca     180

atggtatgga gagatttccc gagacaccaa gaactggaag attatcctgt gtctgtttat     240

tatacccttg gtgggctgtg gctttgtatc atttaggtac aaaccaaggc acataatcgt     300

gtgtgagtgt gtgtgccagt gtgtgtacat gcatccacat atgtgtgctc tcatgtaaat     360

gattaaaaag cctggaactt aaaaaaaaaa a                                    391


<210>  3
<211>  2136
<212>  DNA
<213>  Homo sapiens

<400>  3
ggactacatt attttcactc taagattgat ccacattttt actgtaagca gaaacttagg      60

agccaagatt ataatgctgc agaggatgct gatcgatgtg ttcttcttcc tgttcctctt     120

tgcggtgtgg atggtggcct ttgcgtggcc aggcaaggga tccttaggca gaatgagcag     180

cgctggaggt ggatattccg ttcggtcatc tacgagccct acctggccat gttcggccag     240

gtgcccagtg acgtggatgg taagcctgac ttggctcaga tggaaacagc ttggaggagg     300

catttgctcc ctgaaccaac ccccagggct gccccggaga ccgcacttca gaagcacgcg     360

cgtgaaacgg agtccaacat aacagagtac cacgtatgac tttgcccact gcaccttcac     420

tgggaatgag tccaagccta ctgtgtgtgg agctggatga gcacaacctg ccccggttcc     480

ccgagtggat caccatcccc ctggtgtgca tctacatgtt atccaccaac atcctgctgg     540

tcaacctgct ggtcgccatg tttggctaca cggtgggcac cgtccagaga acaatgacca     600

ggtctggaag ttccagaggt acttcctggt gcaggagtac tgcagccgcc tcaatatccc     660

cttccccttc atcgtcttcg cttacttcta catggtggtg aagaagtgct tcaagtgttg     720

ctgcaaggag aaaaacatgg agtcttctgt ctgctgtttc aaaaatgaag acaatgagac     780

tctggcatgg gagggtgtca tgaaggaaaa ctaccttgtc aagatcaaca caaaagccaa     840

cgacacctca gaggaaatga ggcatcgatt tagacaactg gatacaaagc ttaatgatct     900

caagggtctt ctgaaagaga ttgctaataa aatcaaataa aactgtatga actctaatgg 960

agaaaaatct aattatagca agatcatatt aaggaatgct gatgaacaat tttgctatcg 1020

actactaaat gagagatttt cagacccctg ggtacatggt ggatgatttt aaatcaccct 1080

agtgtgctga gaccttgaga ataaagtgtg tgattggttt catacttgaa gacggatata 1140

aaggaagaat atttccttta tgtgtttctc cagaatggtg cctgtttctc tctgtgtctc 1200

aatgcctggg actggaggtt gatagtttaa gtgtgttctt accgcctcct ttttccttta 1260

atcttatttt tgatgaacac atatatagga gaacatctat cctatgaata agaacctggt 1320

catgctttac tcctgtattg ttattttgtt catttccaat tgattctcta cttttccctt 1380

ttttgtatta tgtgactaat tagttggcat attgttaaaa gtctctcaaa ttaggccaga 1440

ttctaaaaca tgctgcagca agaggacccc gctctcttca ggaaaagtgt tttcatttct 1500

caggatgctt cttacctgtc agaggaggtg acaaggcagt ctcttgctct cttggactca 1560

ccaggctcct attgaaggaa ccacccccat tcctaaatat gtgaaaagtc gcccaaaatg 1620

caaccttgaa aggcactact gactttgttc ttattggata ctcctcttat ttattatttt 1680

tccattaaaa ataatagctg gctattatag aaatttagac catacagaga tgtagaaaga 1740

acataaattg tccccattac cttaaggtaa tcactgctaa caatttctgg atggtttttc 1800

aagtctattt tttttctatg tatgtctcaa ttctctttca aaattttaca gaatgttatc 1860

atactacata tatacttttt atgtaagctt tttcacttag tattttatca aatatgtttt 1920

tattatattc atagccttct taaacattat atcaataatt gcataatagg caacctctag 1980

cgattaccat aattttgctc attgaaggct atctccagtt gatcattggg atgagcatct 2040

ttgtgcatga atcctattgc tgtatttggg aaaattttcc aaggttagat tccaataaat 2100

atctatttat tattcaatat taaaaaaaaa aaaaaa 2136

<210> 4
<211> 1813
<212> DNA
<213> Homo sapiens

<400> 4
   gctagaattt accagtaagc catctgattt cccagtaagc catcctgggc ttttctttgt 60
   tgaaagcttt ttgattgctg attttcattt tcttcatttg ttgtttgtct gttcaggctt 120
   tgtatttctt cttgattcag gtctttgtaa gttgtacatt tctgggatat ttccatttct 180
   tctaggttgt ccaccttgtt tgcatataat tgttcatact agccccttct gatccctttc 240
   atttctatgc cctctgttgt aaggttgtct ttctcatttc tgactgtatt tatttgtatc 300
   ttcttccttt tcttaaaagg tttgttgatt ttgtttatct tttcaaaaaa ccaactctta 360
   ctttcaatga ttttttttcc cattgttttt caactctctt ttttaaaaat gtattttgct 420
   cttggagttt ttgctctact ttaaacagct tactaaagtc attttactat taacaaatac 480
   aaggctcttt caaaagctcc tatagggaat acaaaatttc cccatctcct tataccagaa 540
   aacaaagtta tttacaattc atcttaagtc tcttaatgat ctcaagggtc ttctgaaaga 600
   gattgctaat aaaatcaaat aaaactgtat gaactctaat ggagaaaaat ctaattatag 660
   caagatcata ttaaggaatg ctgatgaaca attttgctat cgactactaa atgagagatt 720
   ttcagacccc tgggtacatg gtggatgatt ttaaatcacc ctagtgtgct gagaccttga 780
   gaataaagtg tgtgattggt ttcatacttg aagacggata taaaggaaga atatttcctt 840
   tatgtgtttc tccagaatgg tgcctgtttc tctctgtgtc tcaatgcctg ggactggagg 900
   ttgatagttt aagtgtgttc ttaccgcctc ctttttcctt taatcttatt tttgatgaac 960
   acatatatag gagaacatct atcctatgaa taagaacctg gtcatgcttt actcctgtat 1020
   tgttattttg ttcatttcca attgattctc tacttttccc ttttttgtat tatgtgacta 1080
   attagttggc atattgttaa aagtctctca aattaggcca gattctaaaa catgctgcag 1140
   caagaggacc ccgctctctt caggaaaagt gttttcattt ctcaggatgc ttcttacctg 1200
   tcagaggagg tgacaaggca gtctcttgct ctcttggact caccaggctc ctattgaagg 1260
   aaccaccccc attcctaaat atgtgaaaag tcgcccaaaa tgcaaccttg aaaggcacta 1320
   ctgactttgt tcttattgga tactcctctt atttattatt tttccattaa aaataatagc 1380
   tggctattat agaaatttag accatacaga gatgtagaaa gaacataaat tgtccccatt 1440
   accttaaggt aatcactgct aacaatttct ggatggtttt tcaagtctat tttttttcta 1500
   tgtatgtctc aattctcttt caaaatttta cagaatgtta tcatactaca tatatacttt 1560
   ttatgtaagc tttttcactt agtattttat caaatatgtt tttattatat tcatagcctt 1620
   cttaaacatt atatcaataa ttgcataata ggcaacctct agcgattacc ataattttgc 1680
   tcattgaagg ctatctccag ttgatcattg ggatgagcat ctttgtgcat gaatcctatt 1740
   gctgtatttg ggaaaatttt ccaaggttag attccaataa atatctattt attattcaat 1800
   attaaaaaaa aaa 1813

<210> 5
<211> 986
<212> DNA
<213> Homo sapiens

<400> 5
acctggctaa tttttgtatt tttagtagac acggggtttc accatgttgg ccaggctggt 60

ctcgaactcc tgacctcagg tgatttgcct gcctcggcct cccaagtgtt gggattacag 120

gcgtgaacca ccgtgtccgg cctcaggttt tcttaattgc agagcttagt gtggtatact 180

ttctgaaggt atctaacagg gaataggggc aaacaaatag ctgcatgctc ctgtcatagt 240

ccaccagcta tgatctgctt aaaacagctg cctgctggtc gccatgtttg gctacacggt 300

gggcaccgtc caggagaaca atgaccaggt ctggaagttc cagaggtact tcctggtgca 360

ggagtactgc agccgcctca atatcccctt ccccttcatc gtcttcgctt acttctacat 420

ggtggtgaag aagtgcttca agtgttgctg caaggagaaa aacatggagt cttctgtctg 480

ctgtttcaaa aatgaagaca atgagactct ggcatgggag ggtgtcatga aagaaaacta 540

ccttgtcaag atcaacacaa aaaccaacga cacctcagag gaaatgaggc atcgatttag 600

acaactggat acaaagatca tattaaggaa tgctgatgaa caattttgct atcgactact 660

aaatgagaga ttttcagacc cctgggtaca tggtggatga ttttaaatca ccctagtgtg 720

ctgagacctt gagaataaag tgtgtgattg gtttcatact tgaagacgga tataaaggaa 780

gaatatttcc tttatgtgtt tctccagaat ggtgcctgtt tctctctgtg tctcaatgcc 840

tgggactgga ggttgatagt ttaagtgtgt tcttaccgcc tcctttttcc tttaatctta 900

tttttgatga acacatatat aggagaacat ctatcctatg aataagaacc tggtcatgct 960

ttaaaaaaaa aaaaaaaaaa aaaaaa 986

<210> 6
<211> 929
<212> DNA
<213> homo sapiens

<400> 6
ggcacgaggc tgcctttctc caccagagac tcttcctcag ggaggacttg gtgaatttta 60

ttcaagcaaa ttttaagaaa cgagaatgtg tcttctttac caaagattcc aaggccacgc 120

tcaatgaaat ccttccttcc tgtccacacc atcgtgctta tcagggagaa tgtgtgcaag 180

tgtggctatg cccagagcca gcacatggaa ggcacccaga tcaaccaaag tgagaaatgg 240

aactacaaga aacacaccaa ggaatttcct accgacgcct ttggggatat tcagtttgag 300

acactgggga agaaagggaa gtatatacgt ctgtcctgcg acacggacgc ggaaatcctt 360

tacgagctgc tgacccagca ctggcacctg aaaacaccca acctggtcat ttctgtgacc 420

gggggcgcca agaacttcgc cctgaagccg cgcatgcgca agatcttcag ccggctcatc 480

tacatcgcgc agtccaaagg tgcttggatt ctcacgggag gcacccatta tggccgatga 540

agtacatcgg ggaggtggtg agagataaca ccatcagcag gagttcagag gagaatattg 600

tggccattgg catagcagct tggggcatgg tctccaaccg ggacaccctc atcaggaatt 660

gcgatgctga ggtaccggtg ggacaggagg aggtctgcta ggtcacatgg aagaaagacc 720

atggcatggg cctgtggcct gaaccctggg gctctgtgat ggagccagcc agatcatggg 780

gaagtctgcc tttcaaggag tgcctttggg accttaaagg aattgaaaac aaggatgacg 840

tacctaatta actgctggga aagagttaac aatgaatgtt ttgttcatta aaatgtgttc 900

tcagcaatct caaaaaaaaa aaaaaaaaa 929

<210> 7
<211> 735
<212> DNA
<213> homo sapiens

<400> 7
ttggccttca gagcaaagaa ggagatctgc atctctacac ccagatggag aatcaccctc 60

actttgcagc tgaaggcaat gtggagttga tgttatttta taccatttat ttttattatc 120

tcttcacaac aaacctacta agtcaatgtt atgattccat gctgcaaaca aggaaattaa 180

gcctcagcaa tcctgatatt ctggaacaga acaatccttt aagagatttg gtattgaaga 240

ccttgttgga aatggatcag acattgccca gaccactgtc cagacccaac actggaataa 300

cccaggagag cttcgtgctt acctcccatc ggcggtcatt ggtgaaaatc tcatcattgg 360

ctaagtccag ctggttccac tccagcagaa gcttcagctg cccattccag ttatccttgt 420

cttgctcact ggtgctgaag gctgtgagag ggcaggaaaa gactcaactc accaaaggct 480

cagaaataag agtgagaacc attcagtgtg gccaattatc agagctgttt atcacagatc 540

gtatttgttc ttaaatggta tctaccagaa gaagacagcc agctttcgat actaacaaac 600

cacaatggaa gatggccgta tttatcattg cctttagcat gttaaagggt acataccaca 660

ttgaccctgg cagaagcatt cctgatgtgt tggaaaaatt aagagaaata acagttcttt 720

ggcaataaaa aaaaa 735

<210> 8
<211> 84
<212> PRT
<213> homo sapiens

<400> 8

Gly Leu Gln Ser Lys Glu Gly Asp Leu His Leu Tyr Thr Gln Met Glu
1               5                   10                  15

Asn His Pro His Phe Ala Ala Glu Gly Asn Val Glu Leu Met Leu Phe
            20                  25                  30

Tyr Thr Ile Tyr Phe Tyr Tyr Leu Phe Thr Thr Asn Leu Leu Ser Gln
        35                  40                  45

Cys Tyr Asp Ser Met Leu Gln Thr Arg Lys Leu Ser Leu Ser Asn Pro
    50                  55                  60

Asp Ile Leu Glu Gln Asn Asn Pro Leu Arg Asp Leu Val Leu Lys Thr
65                  70                  75                  80

Leu Leu Glu Met

<210> 9
<211> 23
<212> DNA
<213> Artificial

<400> 9
catcgtgctt atcagggaga atg 23

<210> 10
<211> 19
<212> DNA
<213> Artificial

<400> 10
tctgggtgcc ttccatgtg 19

<210> 11

<211>  24
<212>  DNA
<213>  Artificial

<400>  11
tgctaggtca catggaagaa agac                                              24


<210>  12
<211>  19
<212>  DNA
<213>  Artificial

<400>  12
tggctggctc catcacaga                                                    19


<210>  13
<211>  28
<212>  DNA
<213>  Artificial

<400>  13
ctggaagatt atcctgtgtc tgtttatt                                          28


<210>  14
<211>  24
<212>  DNA
<213>  Artificial

<400>  14
gtgccttggt ttgtacctaa atga                                              24


<210>  15
<211>  22
<212>  DNA
<213>  Artificial

<400>  15
ttggctcaga tggaaacagc tt                                                22


<210>  16
<211>  18
<212>  DNA
<213>  Artificial

<400>  16
cgcgcgtgct tctgaagt                                                     18


<210>  17
<211>  23

<212>  DNA
<213>  Artificial

<400>  17
ccattgtttt tcaactctct ttt                                              23


<210>  18
<211>  23
<212>  DNA
<213>  Artificial

<400>  18
gaaagagcct tgtatttgtt aat                                              23


<210>  19
<211>  25
<212>  DNA
<213>  Artificial

<400>  19
tcaggttttc ttaattgcag agctt                                            25


<210>  20
<211>  22
<212>  DNA
<213>  Artificial

<400>  20
ggagcatgca gctatttgtt tg                                               22


<210>  21
<211>  23
<212>  DNA
<213>  Artificial

<400>  21
tgcattgtta caggaagtcc ctt                                              23


<210>  22
<211>  18
<212>  DNA
<213>  Artificial

<400>  22
gggagaggac tgggccat                                                    18


<210>  23
<211>  249
<212>  DNA

<213>  Homo sapiens

<400>  23
gtaccggtgg gacaggagga ggtctgctag gtcacatgga agaaagacca tggcatgggc      60

ctgtggcctg aaccctgggg ctctgtgatg gagccagcca gatcatgggg aagtctgcct     120

ttcaaggagt gcctttggga ccttaaagga attgaaaaca aggatgacgt acctaattaa     180

ctgctgggaa agagttaaca atgaatgttt tgttcattaa aatgtgttct cagcaaaaaa     240

aaaaaaaaa                                                             249


<210>  24
<211>  115
<212>  DNA
<213>  Homo sapiens

<400>  24
gtacaaacca aggcacataa tcgtgtgtga gtgtgtgtgc cagtgtgtgt acatgcatcc      60

acatatgtgt gctctcatgt aaatgattaa aaagcctgga acttaaaaaa aaaaa          115


<210>  25
<211>  127
<212>  DNA
<213>  Homo sapiens

<400>  25
gtaagcctga cttggctcag atggaaacag cttggaggag gcatttgctc cctgaaccaa      60

cccccagggc tgccccggag accgcacttc agaagcacgc gcgtgaaacg gagtccaaca     120

taacaga                                                               127


<210>  26
<211>  571
<212>  DNA
<213>  Homo sapiens

<400>  26
gctagaattt accagtaagc catctgattt cccagtaagc catcctgggc ttttctttgt      60

tgaaagcttt ttgattgctg attttcattt tcttcatttg ttgtttgtct gttcaggctt     120

tgtatttctt cttgattcag gtctttgtaa gttgtacatt tctgggatat ttccatttct     180

tctaggttgt ccaccttgtt tgcatataat tgttcatact agccccttct gatccctttc     240

atttctatgc cctctgttgt aaggttgtct ttctcatttc tgactgtatt tatttgtatc     300

ttcttccttt tcttaaaagg tttgttgatt ttgtttatct tttcaaaaaa ccaactctta     360

ctttcaatga ttttttttcc cattgttttt caactctctt ttttaaaaat gtattttgct 420

cttggagttt ttgctctact ttaaacagct tactaaagtc attttactat taacaaatac 480

aaggctcttt caaaagctcc tatagggaat acaaaatttc cccatctcct tataccagaa 540

aacaaagtta tttacaattc atcttaagtc t 571

<210> 27
<211> 271
<212> DNA
<213> Homo sapiens

<400> 27
acctggctaa tttttgtatt tttagtagac acggggtttc accatgttgg ccaggctggt 60

ctcgaactcc tgacctcagg tgatttgcct gcctcggcct cccaagtgtt gggattacag 120

gcgtgaacca ccgtgtccgg cctcaggttt tcttaattgc agagcttagt gtggtatact 180

ttctgaaggt atctaacagg gaataggggc aaacaaatag ctgcatgctc ctgtcatagt 240

ccaccagcta tgatctgctt aaaacagctg c 271

<210> 28
<211> 35
<212> DNA
<213> Homo sapiens

<400> 28
ctgcctttct ccaccagaga ctcttcctca gggag 35

<210> 29
<211> 46
<212> DNA
<213> Homo sapiens

<400> 29
gctcaatgaa atccttcctt cctgtccaca ccatcgtgct tatcag 46

<210> 30
<211> 255
<212> DNA
<213> Homo sapiens

<400> 30
gtaccggtgg gacaggagga ggtctgctag gtcacatgga agaaagacca tggcatgggc 60

ctgtggcctg aaccctgggg ctctgtgatg gagccagcca gatcatgggg aagtctgcct 120

ttcaaggagt gcctttggga ccttaaagga attgaaaaca aggatgacgt acctaattaa 180

ctgctgggaa agagttaaca atgaatgttt tgttcattaa aatgtgttct cagcaatctc 240

aaaaaaaaaa aaaaa 255

<210> 31
<211> 128
<212> DNA
<213> Homo sapiens

<400> 31
tcaggttttc ttaattgcag agcttagtgt ggtatacttt ctgaaggtat ctaacaggga 60

ataggggcaa acaaatagct gcatgctcct gtcatagtcc accagctatg atctgcttaa 120

aacagctg 128